# EUROPEAN PATENT SPECIFICATION

(11) **EP 3 571 180 B1**
(45) Date of publication and mention of the grant of the patent: **18.12.2024**
(21) Application number: 18702635.6
(22) Date of filing: 23.01.2018
(51) Int. Cl.: C07C 37/20, C07C 37/84, C07C 41/30, C07C 41/40, C07C 39/16, C07C 43/23

(54) **PRODUCTION OF HIGHLY PURE META,META-COUPLED BIS(4-ALKYLPHENOL) DERIVATIVES AND USES THEREOF**
HERSTELLUNG VON HOCHREINEN META,META-GEKOPPELTEN BIS(4-ALKYLPHENOL)-DERIVATEN UND VERWENDUNGEN DAVON
PRODUCTION DE DÉRIVÉS DE BIS(4-ALKYLPHÉNOL) MÉTA,MÉTA-COUPLÉS TRÈS PURS ET LEURS UTILISATIONS

(30) Priority: 23.01.2017 GB 201701112; 23.01.2017 GB 201701113
(43) Date of publication of application: 27.11.2019
(73) Proprietor: Katholieke Universiteit Leuven, 3000 Leuven (BE)
(72) Inventor: SELS, Bert, 2260 Westerlo (BE); KOELEWIJN, Steven-Friso, 3000 Leuven (BE)
(74) Representative: IPLodge bv
(86) International application number: PCT/EP2018/051526
(87) International publication number: WO 2018/134427

(56) References cited:
- EP-A1- 0 216 267
- GB-A- 2 339 431
- HEATHER A. MEYLEMANS ET AL: "Synthesis of Renewable Bisphenols from Creosol", CHEMSUSCHEM, vol. 5, no. 1, 9 January 2012 (2012-01-09), pages 206 - 210, XP055200938, ISSN: 1864-5631, DOI: 10.1002/cssc.201100402

## Description

### FIELD OF INVENTION

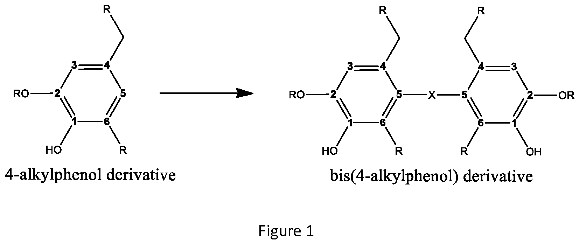

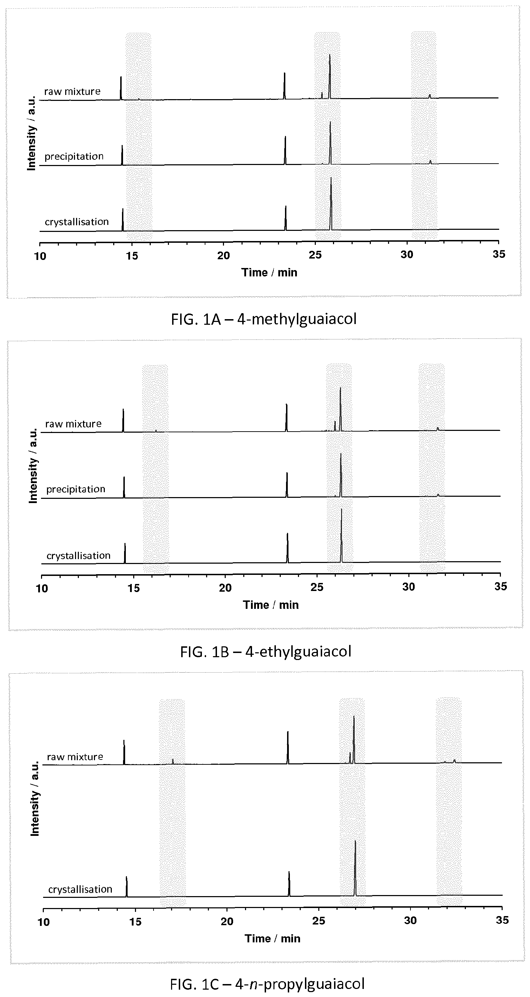

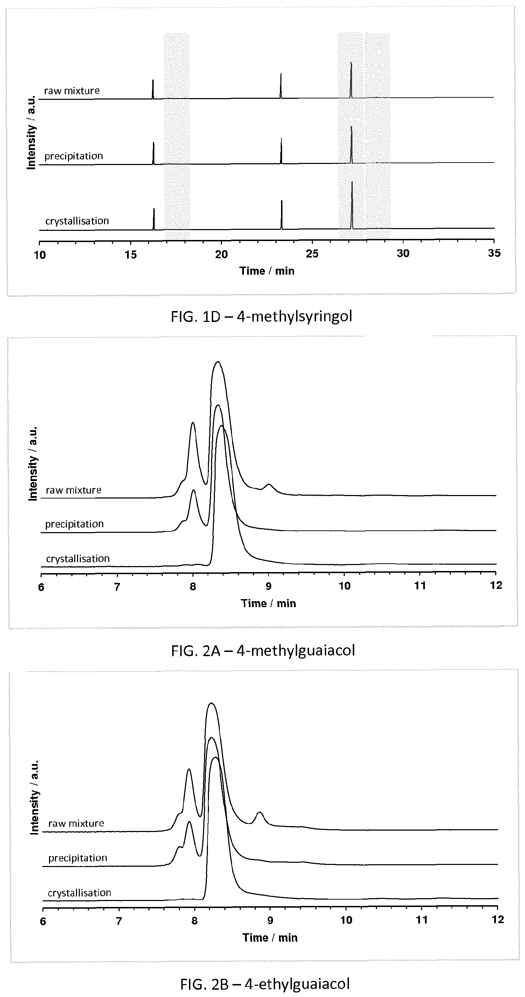

In general the invention relates to a process for production of highly pure meta,meta'-coupled bis(4-alkylphenol) derivatives directly from a raw reaction product mixture in which condensation reactions of 4-alkyl-2-alkoxyphenols (e.g. 2-methoxy-4-*n*-propylphenol) and/or 4-alkyl-2,6-dialkoxyphenols (e.g. 2,6-dimethoxy-4*-n*-propylphenol) have occurred. More specifically this invention relates to a direct process for the selective crystallization of highly pure meta,meta'-coupled bis(4-alkylguaiacol) and/or bis(4-alkylsyringol) derivatives in the presence of residual substrate and isomeric and/or oligomeric by-products/impurities. These high purity bis(4-alkylphenols) (Figure 1) are valuable precursors for renewable thermoplastics and (high temperature) thermosets resins.

### BACKGROUND

Lignocellulose biomass is a highly abundant and potentially low cost renewable feedstock to produce heat, power, fuels, chemicals and materials as an alternative for fossil resources (M. Dusselier, M. Mascal and B. Sels, in Selective Catalysis for Renewable Feedstocks and Chemicals, ed. K. M. Nicholas, Springer International Publishing, 2014, vol. 353, ch. 544, pp. 1-40; J. S. Luterbacher, D. Martin Alonso and J. A. Dumesic, Green Chem., 2014, 16, 4816-4838).

Lignocellulose is composed of three main constituents, cellulose, hemicellulose and lignin. Until recently, lignocellulose biorefineries have mainly focused on the valorization of (hemi)cellulose, while lignin was regarded as a side product and mostly used as energy source (by burning) (J. Zakzeski, et al., Chem Rev., 2010, 110, 3552-3599). Only a minor amount of lignin is used for material applications (dispersants, emulsifiers,...) or in the production of chemicals like vanillin (J. Lora, in Monomers, Polymers and Composites from Renewable Resources, ed. M. N. B. Gandini, Elsevier, Amsterdam, 2008, pp. 225-241; J. Bozell, in Selective Catalysis for Renewable Feedstocks and Chemicals, ed. K. M. Nicholas, Springer International Publishing, 2014, vol. 353, ch. 535, pp. 229-255).

There is however a growing consensus that lignin valorization is essential to the environmental sustainability and economics of a lignocellulosic biorefinery (A. J. Ragauskas, et al., Science, 2014, 344, 709). Lignin constitutes the largest direct source of renewable aromatic/phenolic compounds on Earth and is regarded as a promising feedstock for a wide variety of bulk and fine chemicals, as well as fuels.

The most important lignin depolymerization methods are pyrolysis, base-catalyzed depolymerization, hydrogenolysis, liquid-phase reforming, chemical oxidation and gasification, usually yielding moderate amounts (< 20 wt%) of numerous monomeric compounds (C. Xu, R.A.D. Arancon, J. Labidid and R. Luque, Chem. Soc. Rev. 2014, 43, 7485-7500). Very promising routes for lignin depolymerization are reductive methods like hydrogenolysis and liquid-phase reforming, resulting in phenolic monomer-yields up to 55 wt% (J. M. Pepper and W. Steck, Can. J. Chem., 1963, 41, 2867-2875; J.M. Pepper, P. Supathana, Can. J. Chem., 1978, 56, 899-902; N. Yan, C. Zhao, P. J. Dyson, C. Wang, L. T. Liu and Y. Kou, Chemsuschem, 2008, 1, 626-629; C. Li, M. Zheng, A. Wang and T. Zhang, Energy Environ. Sci., 2012, 5, 6383-6390; Q. Song, F. Wang, J. Y. Cai, Y. H. Wang, J. J. Zhang, W. Q. Yu and J. Xu, Energy Environ. Sci., 2013, 6, 994-1007). The phenolic compounds obtained from these processes possess unique chemical structure features. The phenolic entity bears one or two methoxy groups, next to a 4-alkyl chain. Use of such 4-alkylguaiacols (4-alkyl-2-methoxyphenols) and 4-alkylsyringols (4-alkyl-2,6-dimethoxyphenols) as intermediates for high-value target chemicals and polymer building blocks has thus far received only little attention. Until now, main research goals have focused on upgrading the lignin-derived compounds to hydrocarbon fuels or aromatics by complete removal of oxygen through hydrodeoxygenation (HDO).

A possible processing of the phenolic compounds into useful products is its conversion into alternative bisphenols *(*i*.e. m*,*m'*-bis(4-alkylphenol) derivatives), which maintain both the methoxy group(s) in *ortho* position, and an alkyl moiety in *para* position with respect to the phenolic hydroxyl group. As a tangential benefit, alternative bisphenols may have significantly lower toxicity than typical precursors of bisphenol A. However, a challenge remains the production of highly pure *m*,*m'*-bis(4-alkylphenol) derivatives, since commercial bisphenols, like bisphenol A, are typically required in high purity (≥99.5 wt%) for polycondensation reactions towards polycarbonates (Kumar et al., 2008, US7,371,902 B2). This polycarbonate is known for its superior high-performance properties, including a high transparency, high colour stability, low colour and good melt stability. Moreover, thermoplastic compositions including this polycarbonate resulted in materials with a good impact strength (A. R. Brian, 1987, EP 0216267 A1) and flame retardancy (Cheol et al., 2000, GB2339431B). Development of alternatives to existing polycarbonates that maintain properties are of great interest for both the plastics and manufacturing industry. Hence, alternative high-purity bisphenols are necessary to produce polymeric materials with the desirable properties.

Next, the state-of-the-art concerning the synthesis and purification of m,m'-bis(4-alkylphenol) derivatives is summarized and ordered chronologically.

To initiate a model study of the relationship between structure and antioxidant activity (Pospíšil et *al., Eur. Polym. J.,* 1971, 7, 33-40) Halaska et al., Chemicky Prumysl, 1971, 21, 277-279 prepared a broad collection of methylenebisphenols. Among these also 4-methylphenols (like 2-methoxy-4-methylphenol, 2-ethoxy-4-methylphenol and 4-methyl-1,2-benzenediol) were used. Thereto, a stoichiometric amount of paraformaldehyde was added to a solution of the phenol in formic acid (1 g in 1 mL acid) and the mixture heated at 110 °C for 20 minutes. The isolated products, being 5,5'-methylenebis(2-methoxy-4-methylphenol), 5,5'-methylenebis(2-ethoxy-4-methylphenol) and 5,5'-methylenebis(4-methyl-1,2-benzenediol), were obtained, after purification of the reaction mixture by a combination of extraction, column chromatography (silica gel impregnated with formamide) and repeated crystallization, in 37 (benzene-hexane), 32 (ethyl acetate-hexane) and 25 wt% (ether-benzene), with melting points at 139, 127 and 144-145 °C respectively. Higher unidentified condensation product of the starting phenols 2-methoxy-4-methylphenol and 4-methyl-1,2-benzenediol were isolated in only 2.6 wt% and 7.5 wt% only. The multi-step purification, with the need for column chromatography, limits an industrial implementation. Moreover, the use of controversial solvents like benzene and hexane are preferably avoided.

In their study of lignin model compounds Kratzl and Wagner, Holzforsch. Holzverwert., 1972, 24, 56-61 coupled creosol (i.e. 2-methoxy-4-methylphenol) with formaldehyde in 2N HCl/dioxane and also obtained 5,5'-methylenebis(2-methoxy-4-methylphenol). Thin layer chromatography revealed that higher condensation products were present only in small amounts. The isolated product was obtained in <48 wt% only after purification of the reaction mixture (being a brown powder) by column chromatography (silica gel/benzene). Recrystallizations of the pure product from benzene eventually gave crystals with a melting point around 136-138 °C. Said process is unwanted for above mentioned reasons.

Ito et al., Mokuzai Gakkaishi, 1981, 27, 484-90 treated different arylglycerol-β-aryl ethers, as main structural unit in lignin, with 5% H₂SO₄, which resulted in the formation of 5,5'-methylenebis(2-methoxy-4-methylphenol). These results indicated that formation of diarylmethane structures occur during the hydrolysis of wood or lignin with H₂SO₄. Besides ia *ortho*,*meta*'-coupled dimer *(i.e.* 5-(2-hydroxy-3-methoxy-5-methylbenzyl)-2-methoxy-4-methylphenol).

In order to elucidate the behavior of syringylglycerol-β-syringyl ether as a main structural unit in angiosperm lignin under the influence of sulfuric acid, Yasuda and Ota, Holzforschung, 1987, 41, 59-65 treated phenolic and non-phenolic model compounds in dilute (5%) and concentrated sulfuric acid (72%). Reaction of both non-phenolic and phenolic syringylglycerol-β-syringyl ether in both 5% and 72% sulfuric acid yielded 5,5'-methylenebis(2,6-dimethoxy-4-methylphenol). The isolated product was obtained in 7.3-29.4 wt% after purification of the product mixture by column chromatography (on silica gel with n-hexane/acetone). The compound melted around 146-147 °C. The formation of this dimer suggests elimination of the hydroxymethyl group as formaldehyde to some extent, which undergoes acid-promoted condensation with liberated and/or added 2,6-dimethoxy-4-methylphenol. Said process is unwanted for above mentioned reasons.

Inatomi et al., 1995, JP 07089888A reacted 4-methylcatechol with an aldehyde in the presence of an acid catalyst to form 5,5'-methylenebis(4-methyl-1,2-benzenediol). High purity biscatechol was prepared in high yield, and the biscatechol was useful for positive working photoresist. No details about purification procedure can be found.

Meylemans et al., ChemSusChem, 2012, 5(1), 206-210 reported a series of renewable bisphenols synthesized from creosol through stoichiometric condensation with short shain aldehydes (like formaldehyde, acetaldehyde and propionaldehyde) in aqueous acidic (i.e. HCl, HBr) media. The isolated products, being 5,5'-methylenebis(2-methoxy-4-methylphenol), 5,5'-(ethane-1,1-diyl)bis(2-methoxy-4-methylphenol) and 5,5'-(propane-1,1-diyl)bis(2-methoxy-4-methylphenol), were obtained as white solids in only 63, 67 and 68 wt% respectively, after dissolving in ether and precipitating with heptane. The first two compounds melted around 131-134 and 143-146 °C. Although ortho,meta'-isomers were detected, no details were given on their fate during precipitation. Neither a report is given on the formation of higher condensation products (i.e. oligomers, such as trimers). Subsequently, Meylemans et al., Biomacromolecules, 2013, 14, 771-780 converted these bisphenols to a series of renewable bis(cyanate) esters (in 46-96 wt%) with applications in a wide variety of military and commercial applications. The bisphenols can also be applied in organic thin film transistors having solution-processed n-type copolymers as the semiconductor material (Baca et al., 2015, US 9,082,983 B1).

Chen et al, ChemCatChem, 2015, 7, 1083-1089 also performed the synthesis of creosol-based bisphenols in water. Lignosulfonic acid (20 mol%), a byproduct of the paper industry, was demonstrated to be an efficient and green catalyst in water for this stoichiometric condensation of creosol with formaldehyde. The isolated product, being 5,5'-methylenebis(2-methoxy-4-methylphenol), was obtained in only 52.7 wt% after purification of the reaction products by column chromatography (petroleum ether/ethyl acetate = 3:1) and melted at 134-136 °C. Also the o,m'-isomer and a trimeric species were isolated (in 2.5 wt% and 3.6 wt% respectively) and characterized for the first time. Hereafter, this bisphenol was converted into polycarbonates (in 83% yield) and polyurethanes by polyaddition of bis(cyclic carbonate) and diamines (Chen et al., Green Chem., 2015, 17, 4546-4551).

Van der Klashorst and Strauss, J. Polym. Sci. A Polym. Chem., 1986, 24, 2143-2169 describe the synthesis of 5,5'-methylenebis(4-ethyl-2-methoxyphenol) and 5,5'-methylenebis(4-ethyl-2,6-dimethoxyphenol) from respectively 4-ethyl-2-methoxyphenol and 4-ethyl-2,6-dimethoxyphenol with 20 fold excess formaldehyde in 0.82 M HCl in 50 % aqueous dioxane at ambient temperature for 48 and 18 h respectively. The isolated products were obtained in only 48.1 wt% and 67.0 wt% respectively after purification of the crude product by flash chromatography (silica gel with hexane/ethyl acetate = 2:1). Besides the main product, in the case of the guaiacyl analog, also mono-hydroxymethylated monomers (15 wt%) and dimers (21 wt%), and trimers (15 wt%) were isolated. In the case of the syringyl analog, considerable amounts of trimers (19.1 wt%) formed. When the same reactions were performed with an equimolar quantity of formaldehyde in 2.2 M HCl in 50% aqueous dioxane at 80 °C a maximum yield of 83 % and 78 % (as determined by HPLC) were reached within 1.5-2 and 0.3-0.4 h respectively. In these conditions, considerable amount of higher condensation products (tri-, tetra- and oligomers) were observed in the case of the syringyl analog. The same chemistry yields lignin meta-hydroxyalkyl derivatives by treating lignin with a reagent (e.g. an aldehyde) in an acid medium in the presence of an organic solvent. The substituted lignin may be reacted with a nucleophile. Thus, alkali lignin from different industrial origins reacted with formaldehyde in 50% aqueous dioxane under acidic conditions at positions 2- and 6- gave methylene crosslinks which resulted in the formation of a polymeric product, whereas using 10% aqueous dioxane and 0.4-1N HCl, *meta*-hydroxymethylation was the preferential reaction (Van der Klashorst, In: Lignin, Ch. 26, 1989, 346-360; Van der Klashorst, 1989, ZA 198805771 A).

From the above-mentioned state-of-the-art, based on the literature melting points reported for identical compounds, one skilled in the art can conclude that residual impurities often remain, as for example in the case of 5,5'-methylenebis(2-methoxy-4-methylphenol).

Hence, there is a great incentive to produce high-purity *m*,*m'*-coupled bis(4-alkylguaiacol) and bis(4-alkylsyringol) derivatives in high purity (and yield) which can be easily purified from the reaction product mixture containing residual substrate, (*m*,*m'*- and *o*,*m'*-)dimers and oligomeric by-products/impurities, without the need for solvent-intense methods like column/flash chromatography and the use of controversial solvents (like hexane, benzene and dioxane).

The present invention provides such production of *m*,*m'*-bis(4-alkylguaiacol) and *m*,*m'*-bis(4-alkylsyringol) derivatives in high product yield (57-75 wt%) which is purified from the raw reaction product mixture containing residual substrate, and isomeric (*i.e*. *o*,*m'*-product) and/or oligomeric by-products/impurities with high purity of ≥99.0 % of *m*,*m'*-coupled bis(4-alkylguaiacol) or bis(4-alkylsyringol) derivative by wt. of the end-product, more preferably ≥99.5 % of *m*,*m'*-coupled bis(4-alkylguaiacol) and bis(4-alkylsyringol) derivative by wt. of the end-product as analysed by melting point measurement (by differential scanning calorimetry), gas chromatography and/or gel-permeation/size-exclusion chromatography.

### SUMMARY OF THE INVENTION

The present invention anticipates on the future utilization of various 4-alkylphenol derivatives (e.g. 2-methoxy-4-*n*-propylphenol and 2,6-dimethoxy-4-*n*-propylphenol) for synthesis of alternative bisphenols. These 4-alkylphenol derivatives can be obtained from, among other, the depolymerisation of lignin enclosed in lignocellulosic biomass.

From raw reaction product mixtures in which condensation reactions of 4-alkyl-2-alkoxyphenols (e.g. 2-methoxy-4-*n*-propylphenol) or 4-alkyl-2,6-dialkoxyphenols (e.g. 2,6-dimethoxy-4-*n*-propylphenol) have occurred, *m*,*m'*-coupled bis(4-alkylphenol) derivatives are selectively crystallized in high purity of at least 99 % of *m*,*m'*-bis(4-alkylphenol) by wt. of the end-product.

The process of the present invention is useful for the production of high-purity *m*,*m'*-coupled bis(4-alkylguaiacol) and/or bis(4-alkylsyringol), in which the alkyl substituent is for instance methyl, ethyl or n-propyl.

According to the present invention, there is provided a process for the production of aromatic dihydroxy compounds of >72 wt% purity, as defined in claim 1. Optional features of the invention are defined in dependent claims 2-15.

### BRIEF DESCRIPTION OF THE FIGURES

The accompanying figures, which are incorporated in and constitute a part of this specification, illustrate several aspects and together with the description serve to explain the principles of the invention.
FIGS. **1**A, **1**B, **1**C and **1**D show representative GC data of bis(4-alkylphenol) derivatives from respectively 4-methyl-, 4-ethyl-, 4-*n*-propylguaiacol and 4-methylsyringol as present in the condensation mixture (top), as prepared by precipitation (middle) and according to the present invention (bottom).

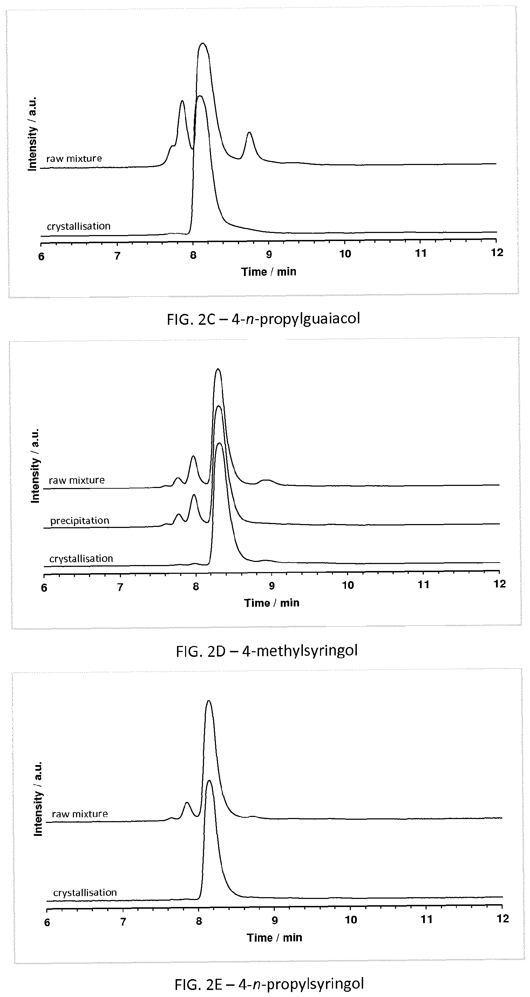

FIGS. **2**A, **2**B, **2**C, **2**D and **2**E show representative GPC/SEC data of bis(4-alkylphenol) derivatives from respectively 4-methyl-, 4-ethyl-, 4-n-propylguaiacol and 4-methyl- and 4-n-syringol as present in the condensation mixture (top), as prepared by precipitation (middle) and according to the present invention (bottom).

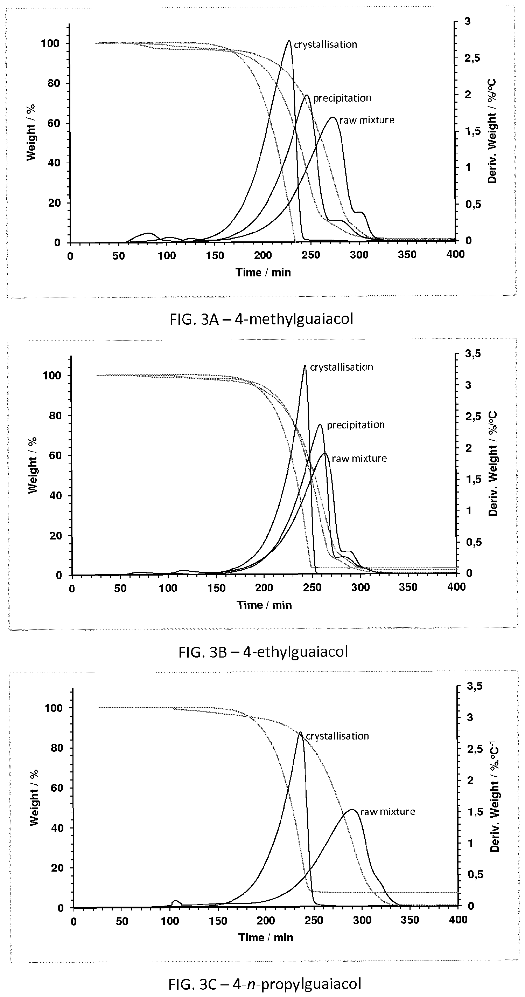

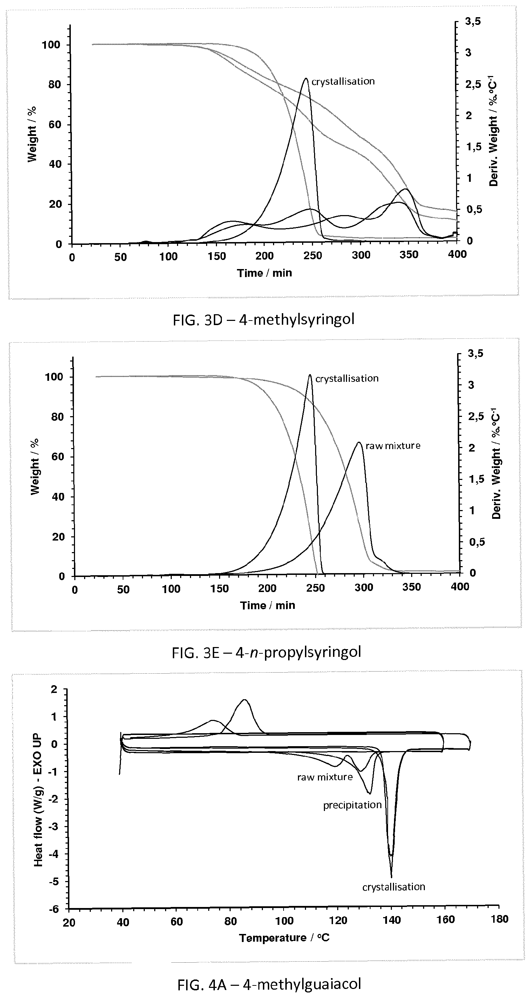

FIGS. **3**A, **3**B, **3**C, **3**D and **3**E show representative thermogravimetric analysis (TGA) data for bis(4-alkylphenol) derivatives from respectively 4-methyl, 4-ethyl-, 4-*n*-propylguaiacol and 4-methyl and 4-n-propylsyringol as present in the condensation mixture (right), as prepared by precipitation (middle) and according to the present invention (left).

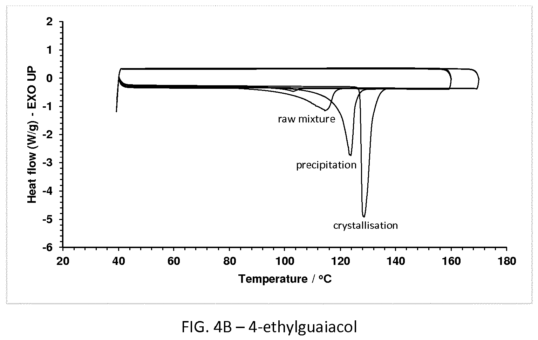

FIGS. **4**A, **4**B show representative differential scanning calorimetry (DSC) data for bis(4-alkylphenol) derivatives from respectively 4-methyl- and 4-ethylguaiacol as present in the condensation mixture (left), as prepared by precipitation (middle) and according to the present invention (right).

### DETAILED DESCRIPTION OF THE INVENTION

The present disclosure can be understood more readily by reference to the following detailed description of the disclosure and the EXAMPLES include therein.

Before the present compounds, compositions, articles, systems, devices, and/or methods are disclosed and described, it is to be understood that they are not limited to specific synthetic methods unless otherwise specified, or to particular reagents unless otherwise specified, as such can, of course, vary. It is also to be understood that the terminology used herein is for the purpose of describing particular aspects only and is not intended to be limiting. Although any methods and materials similar or equivalent to those described herein can be used in the practice or testing the present disclosure, example methods and materials are now described.

Moreover, it is to be understood that unless otherwise expressly stated, it is in no way intended that any method set forth herein be construed as requiring that its steps be performed in a specific order. Accordingly, where a method claim does not actually recite an order to be followed by its steps or it is not otherwise specifically stated in the claims or descriptions that the steps are to be limited to a specific order, it is no way intended that an order be inferred, in any respect. This holds for any possible non-express basis for interpretation, including: matters of logic with respect to arrangement of steps or operational flow; plain meaning derived from grammatical organization or punctuation; and the number or type of embodiments described in the specification.

### A. GENERAL DEFINITIONS

It is also to be understood that the terminology used herein is for the purpose of describing particular aspects only and is not intended to be limiting. As used in the specification and in the claims, the term "comprising" can include the aspects "consisting of" and "consisting essentially of". Unless defined otherwise, all technical and scientific terms used herein have the same meaning as commonly understood by one of ordinary skill in the art to which this invention belongs. In this specification and in the claims which follow, reference will be made to a number of terms which shall be defined herein.

As used in the specification and the appended claims, the singular forms "a," "an" and "the" include plural referents unless the context clearly dictates otherwise. Thus, for example, reference to "a polymer" includes mixtures of two or more polymers.

As used herein, the term "combination" is inclusive of blends, mixtures, alloys, reaction products, and the like.

Ranges can be expressed herein as from one particular value, and/or to another particular value. When such a range is expressed, another aspect includes from the one particular value and/or to the other particular value. Similarly, when values are expressed as approximations, by use of the antecedent 'about,' it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "about" and "at or about" mean that the amount or value in question can be the value designated some other value approximately or about the same. It is generally understood, as used herein, that it is the nominal value indicated ±10% variation unless otherwise indicated or inferred. The term is intended to convey that similar values promote equivalent results or effects recited in the claims. That is, it is understood that amounts, sizes, formulations, parameters, and other quantities and characteristics are not and need not be exact, but can be approximate and/or larger or smaller, as desired, reflecting tolerances, conversion factors, rounding off, measurement error and the like, and other factors known to those of skill in the art. In general, an amount, size, formulation, parameter or other quantity or characteristic is "about" or "approximate" whether or not expressly stated to be such. It is understood that where "about" is used before a quantitative value, the parameter also includes the specific quantitative value itself, unless specifically stated otherwise.

As used herein, the terms "optional" or "optionally" means that the subsequently described event or circumstance can or cannot occur, and that the description includes instances where said event or circumstance occurs and instances where it does not. For example, the phrase "optionally substituted alkyl" means that the alkyl group can or cannot be substituted and that the description includes both substituted and unsubstituted alkyl groups.

As used herein, the term "effective amount" refers to an amount that is sufficient to achieve the desired modification of a physical property of the composition or material. For example, an "effective amount" of a filler refers to an amount that is sufficient to achieve the desired improvement in the property modulated by the formulation component, e.g. achieving the desired level of modulus. The specific level in terms of wt % in a composition required as an effective amount will depend upon a variety of factors including the amount and type of polycarbonates, amount and type of thermally conductive filler, and use of the article made using the composition.

Disclosed are the components to be used to prepare the compositions of the invention as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds cannot be explicitly disclosed, each is specifically contemplated and described herein. For example, if a particular compound is disclosed and discussed and a number of modifications that can be made to a number of molecules including the compounds are discussed, specifically contemplated is each and every combination and permutation of the compound and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the compositions of the disclosure. Thus, if there are a variety of additional steps that can be performed it is understood that each of these additional steps can be performed with any specific aspect or combination of aspects of the methods of the disclosure.

References in the specification and concluding claims to parts by weight of a particular element or component in a composition or article, denotes the weight relationship between the element or component and any other elements or components in the composition or article for which a part by weight is expressed. Thus, in a compound containing 2 parts by weight of component X and 5 parts by weight component Y, X and Y are present at a weight ratio of 2:5, and are present in such ratio regardless of whether additional components are contained in the compound.

As used herein the terms "weight percent," "wt %," and "wt. %," which can be used interchangeably, indicate the percent by weight of a given component based on the total weight of the composition, unless otherwise specified. That is, unless otherwise specified, all wt % values are based on the total weight of the composition. It should be understood that the sum of wt % values for all components in a disclosed composition or formulation are equal to 100.

Compounds are described using standard nomenclature. For example, any position not substituted by any indicated group is understood to have its valence filled by a bond as indicated, or a hydrogen atom. A dash ("-") that is not between two letters or symbols is used to indicate a point of attachment for a substituent. For example, -CHO is attached through carbon of the carbonyl group. Unless defined otherwise, technical and scientific terms used herein have the same meaning as is commonly understood by one of skill in the art to which this disclosure belongs.

The term "alkyl group" as used herein is a branched or unbranched saturated hydrocarbon group of 1 to 24 carbon atoms, such as methyl, ethyl, *n*-propyl, isopropyl, *n*-butyl, isobutyl, *t-*butyl, pentyl, hexyl, heptyl, octyl, decyl, tetradecyl, hexadecyl, eicosyl, tetracosyl and the like. A "lower alkyl" group is an alkyl group containing from one to six carbon atoms.

The term "aryl group" as used herein is any carbon-based aromatic group including, but not limited to, benzene, naphthalene, etc. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulphur, and phosphorus. The aryl group can be substituted or unsubstituted. The aryl group can be substituted with one or more groups including, but not limited to, alkyl, alkynyl, alkenyl, aryl, halide, nitro, amino, ester, ketone, aldehyde, hydroxy, carboxylic acid, or alkoxy.

The term "aralkyl" as used herein is an aryl group having an alkyl, alkynyl, or alkenyl group as defined above attached to the aromatic group. An example of an aralkyl group is a benzyl group.

The term "organic residue" defines a carbon containing residue, i.e., a residue comprising at least one carbon atom, and includes but is not limited to the carbon-containing groups, residues, or radicals defined hereinabove. Organic residues can contain various heteroatoms, or be bonded to another molecule through a heteroatom, including oxygen, nitrogen, sulphur, phosphorus, or the like. Examples of organic residues include but are not limited to alkyl or substituted alkyls, alkoxy or substituted alkoxy, mono or di-substituted amino, amide groups, etc. Organic residues can preferably comprise 1-18 carbon atoms, 1-15 carbon atoms, 1-12 carbon atoms, 1-8 carbon atoms, 1-6 carbon atoms, or 1-4 carbon atoms. In a further aspect, an organic residue can comprise 2-18 carbon atoms, 2-15 carbon atoms, 2-12 carbon atoms, 2-8 carbon atoms, 2-6 carbon atoms, or 2-4 carbon atoms.

A very close synonym of the term "residue" is the term "radical", which as used in the specification and concluding claims, refers to a fragment, group, or substructure of a molecule described herein, regardless of how the molecule is prepared. For example, a 2,4-dihydroxyphenyl radical in a particular compound has the structure: 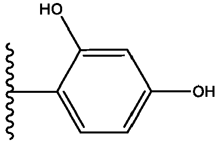 regardless of whether 2,4-dihydroxyphenyl is used to prepare the compound. In some aspects the radical (for example an alkyl) can be further modified (i.e., substituted alkyl) by having bonded thereto one or more "substituted radicals". The number of atoms in a given radical is not critical to the present disclosure unless it is indicated to the contrary elsewhere herein.

"Organic radicals", as the term is defined and used herein, contain one or more carbon atoms. An organic radical can have, for example, 1-26 carbon atoms, 1-18 carbon atoms, 1-12 carbon atoms, 1-8 carbon atoms, 1-6 carbon atoms, or 1-4 carbon atoms. In a further aspect, an organic residue can comprise 2-26 carbon atoms, 2-18 carbon atoms, 2-12 carbon atoms, 2-8 carbon atoms, 2-6 carbon atoms, or 2-4 carbon atoms. Organic radicals often have hydrogen bound to at least some of the carbon atoms of the organic radical. One example, of an organic radical that comprises no inorganic atoms is a 5,6,7,8-tetrahydro-2-naphtyl radical. In some aspects, an organic radical can contain 1-10 inorganic heteroatoms bound thereto or therein, including halogens, oxygen, sulphur, nitrogen, phosphorus, and the like. Examples of organic radicals include but are not limited to an alkyl, substituted alkyl, cycloalkyl, substituted cycloalkyl, mono-substituted amino, di-substituted amino, acyloxy, cyano, carboxy, carboalkoxy, alkylcarboxamide, substituted alkylcarboxamide, dialkylcarboxamide, substituted dialkylcarboxamide, alkylsulfonyl, alkylsulfinyl, thioalkyl, thiohaloalkyl, alkoxy, substituted alkoxy, haloalkyl, haloalkoxy, aryl, substituted aryl, heteroaryl, heterocyclic, or substituted heterocyclic radicals, wherein the terms are defined elsewhere herein. A few nonlimiting examples of organic radicals that include heteroatoms include alkoxy radicals, trifluoromethoxy radicals, acetoxy radicals, dimethylamino radicals and the like.

As used herein, "polycarbonate" refers to an oligomer or polymer comprising residues of one or more dihydroxy compounds, e.g., dihydroxy aromatic compounds, joined by carbonate linkages; it also encompasses homopolycarbonates, copolycarbonates and (co)polyester carbonates.

The terms "residues" and "structural units", used in reference to the constituents of the polymers, are synonymous throughout the specification.

As used herein, "product yield" is defined as the molar amount of product formed per molar amount of starting substrate and "substrate conversion" is defined as the molar amount of substrate converted per molar amount of starting material. "product selectivity" is defined as the ratio of the product yield over the substrate conversion. For instance a particular embodiment context "conversion" means the mole percent of the initial 4-alkylphenol derivative that is reacted to form a different compound, not necessarily all the desired product, while "yield" is the mole percent of the converted starting material that forms any bis(4-alkylphenol) derivative and "selectivity" is the mole percent of the reaction product formed that is bis(4-alkylphenol) derivative. For example, if 10 moles of 4-alkylphenol derivative are reacted and 2 moles of 4-alkylphenol derivative are left unchanged, then the conversion is 80 percent. If the reaction product contains 3 moles of bis(4-alkylphenol) derivative then the yield is 60 percent and the selectivity is 75 percent.

Each of the materials disclosed herein are either commercially available and/or the methods for the production thereof are known to those of skill in the art.

### B. META,META-COUPLED BIS(4-ALKYLPHENOL) DERIVATIVES

In accordance with the purpose(s) of the disclosure, as embodied and broadly described herein, the disclosure, in one aspect, relates to meta,meta'-coupled bis(4-alkylphenol) derivatives which are valuable precursors for renewable thermoplastics, (high temperature) resins/thermosets, bio-derived C15-C19 fuel additives, antioxidants, UV-stabilizers, plasticizers, soluble n-type perylene copolymers and/or positive working photoresists.

In various aspects, the present disclosure relates to a process for purification of meta,meta'-coupled bis(4-alkylphenol) derivatives in high purity. The terms "meta,meta'-coupled bis(4-alkylphenol) derivatives", "m,m'-coupled bis(4-alkylphenol) derivatives", "m,m'-bis(4-alkylphenol) derivatives", "m,m'-coupled dimers" and "m,m'-dimers" which can be used interchangeably, as used herein refers to a compound having a structure represented by the formula:

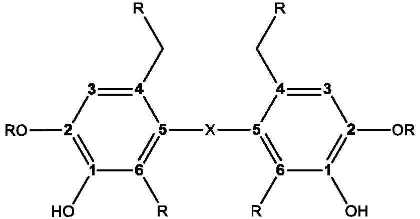

In various aspects, m,m'-bis(4-alkylphenol) derivatives are defined as structures comprising two phenolic units coupled by a one atom bridging group (-X-) connecting the two hydroxyl-substituted aromatic groups selected from oxy (-O-), thio (-S-), sulfinyl (-S(O)-), sulfonyl (-S(O)2-), carbonyl (-C(O)-), or a alkanediyl C1-C24 organic group; and wherein the bridging group (-X-) and the hydroxyl substituent are disposed *meta* to each other on the arylene group bearing the phenolic (Ar-OH) functionality; and wherein the alkyl chain (-CH₂R) and the hydroxyl substituent are disposed *para* to each other on the arylene group bearing the phenolic (Ar-OH) functionality; and wherein at least one alkoxy group (Ar-OR and/or Ar-R) is disposed *ortho* to the arylene groups bearing the phenolic (Ar-OH) functionality.

In various aspects, this general class of monomers is known as aromatic dihydroxy compounds.

### C. THE RAW REACTION PRODUCT MIXTURE

As used herein, "raw reaction product mixture" concerns the rough, crude, non-fractionated, mixed, complex organic phase resulting from the condensation reaction of the 4-alkylphenol derivative, without prior purification steps (such as column chromatography, extraction, and the like). The raw reaction product mixture contains at least residual substrate, dimers and oligomers.

### Condensation reaction

As used herein, "condensation reaction" is defined as and comprises: a) providing a 4-alkylphenol derivative; b) providing a carbonylic compound; c) reacting the phenol and carbonylic compound under conditions effective to provide a reaction product comprising a m,m'-coupled bis(4-alkylphenol) derivative.

In further aspects, conditions effective comprise reacting the 4-alkylphenol derivative and carbonylic compound in the presence of a catalyst. In yet further aspects, the catalyst comprises a strongly acidic catalyst, more preferable a soluble strongly acidic catalyst and even more preferable a water-soluble strongly acidic catalyst. In yet further aspects the water-soluble strongly acidic catalyst comprises a i) hydrohalic acid/hydracid (i.e. hydrochloric, hydrobromic, hydroiodic and/or hydroastatic acid), ii) oxyacid (i.e. nitric, sulphuric and/or perchloric acid) and/or iii) an organic acid, such as (un)halogenated sulfonic acid and halogenated carboxylic acid, and/or mixtures and/or combinations thereof.

In further aspects, the carbonylic compound comprises R(H)(C=O) (i.e. aldehydes), (H)2(C=O)2 (i.e. glyoxal), (H)(C=O)R(C=O)(H) (i.e. dialdehydes), (H)(C=O)(COOH) (i.e. glyoxylic acid), (H)(C=O)(COOR) (i.e. alkyl glyoxylates), R(R)(C=O) (i.e. ketones), R(R)(C=O)2 (i.e. vicinal diketones), (R)(C=O)(COOH) (i.e. α-keto acids) and/or (R)(C=O)(COOR) (i.e. α-keto esters) with R a C1-C24 organic group. In still further aspects the aldehyde is methanal (i.e. formaldehyde), ethanal (i.e. acetaldehyde), propanal (i.e. propionaldehyde), butanal (i.e. butyraldehyde). In still further aspects, the aldehyde is preferably methanal. In still further aspects the dialdehyde is ethanedial (i.e. glyoxal), propanedial (i.e. malonaldehyde), butanedial (i.e. succinaldehyde), pentanedial (i.e. glutaraldehyde), benzene-1,2-carbaldehyde (i.e. phthalaldehyde). In still further aspects the dialdehyde is preferably glyoxal. In still further aspects, the alkyl glyoxalate is methyl glyoxalate, ethyl glyoxalate, propyl glyoxalate. In still further aspects, the alkyl glyoxalate is preferably methyl glyoxalate. In still further aspects the ketone is a methyl ketone, such as acetone, 2-butanone and 2-pentanone. In still further aspects, the ketone is preferably acetone. In still further aspects the vicinal diketone is 2,3-butanedione, 2,3-pentanedione, 2,3-hexanedione or 2,3-heptanedione. In still further aspects, the vicinal diketone is preferably 2,3-butanedione. In still further aspects the α-keto acid is pyruvic acid. In still further aspects the α-keto esters is methyl pyruvate.

In further aspects, conditions effective comprise adjusting the temperature to at least about 20 °C. In still further aspects, the temperature is adjusted to a temperature of from 50 to about 150 °C, including exemplary temperatures of about 55, 60, 65, 70, 75, 80, 85, 90, 95, 100, 105, 110, 115, 120, 125, 130, 135, 140, and 145 °C.

In further aspects, conditions effective comprise maintaining the reaction for at least about 2 hours. In yet further aspects, the reaction is maintained for about 1-20 hours, including exemplary times of 2, 3, 4, 5, 6, 7, 8, 9, 10, 11, 12, 13, 14, 15, 16, 17, 18, and 19 hours.

In further aspects, conditions effective comprise a continuous stirring regime i) in which the contact interface between the liquid aqueous phase and the liquid organic phase is maximal and ii) in which homogeneity conditions (like temperature, concentration) are guaranteed as much as possible.

In various aspects, conditions effective comprise a liquid aqueous phase from at least about 1 wt. % to about 100 wt. % of distilled, de-ionized and/or demineralized water and/or mixtures and/or combinations thereof, including exemplary values of 20 wt%, 25 wt%, 30 wt%, 35 wt%, 40 wt%, 45 wt%, 50 wt%, 55 wt%, 60 wt%, 65 wt%, 70 wt%, 75 wt%, 80 wt%, 85 wt%, 90 wt%, and 95 wt%. In yet further aspects the liquid aqueous phase contains a co-solvent(s) in a range from 0 - 99 wt%, preferably between 0-40 wt%, and even more preferably between 0-15 wt%.

In various aspects, conditions effective comprise the reaction to take place under a (partial) modified or unmodified atmosphere. In some aspects the modified atmosphere comprises the use of inert gasses like nitrogen (N₂), argon (Ar) and/or mixtures and/or combinations thereof.

In some aspects, the 4-alkylphenol derivative is provided as a pure substance to yield symmetric m,m'-bis(4-alkylphenol) derivatives. In some aspects, the 4-alkylphenol derivative consists of mixtures and/or combinations of 2 or more different types to yield asymmetric m,m'-bis(4-alkylphenol) derivatives.

In various aspects, the 4-alkylphenol derivative and aldehyde or ketone are provided at a molar ratio of at least about 2:1. In further aspects, the 4-alkylphenol derivative and aldehyde or ketone are provided at a stoichiometric molar ratio of 2:1.

### residual substrate

As used herein, the term "residual substrate" refers to unreacted, unconverted 4-alkylphenol derivatives.

In further aspects, the 4-alkylphenol derivative comprises 4-alkyl-2-hydroxyphenols (*i.e. 4-*alkylcatechols such as, 2-hydroxy-4-methylphenol, 4-ethyl-2-hydroxyphenol, 2-hydroxy-4-n-propylphenol, 4-n-butyl-2-hydroxyphenol, 2-hydroxy-4-*n*-pentylphenol), 2-alkoxy-4-alkylphenols (*i.e.* 4-alkylguaiacols such as, 2-methoxy-4-methylphenol, 4-ethyl-2-methoxyphenol, 2-methoxy-4-*n*-propylphenol, 4-*n*-butyl-2-methoxyphenol, 2-methoxy-4-*n-*pentylphenol and 4-alkylguethols such as, 2-ethoxy-4-methylphenol, 2-ethoxy-4-ethylphenol, 2-ethoxy-4-n-propylphenol, 4-*n*-butyl-2-ethoxyphenol, 2-ethoxy-4-n-pentylphenol), 4-alkyl-2,6-dihydroxyphenol or 5-alkylbenzene-1,2,3-triol (*i.e*. 5-alkylpyrogallol such as, 5-methylbenzene-1,2,3-triol, 5-ethylbenzene-1,2,3-triol, 5-n-propylbenzene-1,2,3-triol, 5-*n-*butylbenzene-1,2,3-triol, 5-*n*-pentylbenzene-1,2,3-triol), 3-alkoxy-5-alkylbenzene-1,2-diol (*i.e*. 3-alkoxy-5-alkylcatechol such as, 3-methoxy-5-methylbenzene-1,2-diol, 5-ethyl-3-methoxybenzene-1,2-diol, 3-methoxy-5-n-propylbenzene-1,2-diol, 5-n-butyl-3-methoxybenzene-1,2-diol, 3-methoxy-5-*n*-pentylbenzene-1,2-diol, 3-ethoxy-5-methylbenzene-1,2-diol, 3-ethoxy-5-ethylbenzene-1,2-diol, 3-ethoxy-5-n-propylbenzene-1,2-diol, 5-*n*-butyl-3-ethoxybenzene-1,2-diol, 3-ethoxy-5-*n*-pentylbenzene-1,2-diol) and/or 2,6-dialkoxy-4-alkylphenols (*i.e.* 4-alkylsyringol such as, 2,6-dimethoxy-4-methylphenol, 4-ethyl-2,6-dimethoxyphenol, 2,6-dimethoxy-4-*n*-propylphenol, 4-*n*-butyl-2,6-dimethoxyphenol, 2,6-dimethoxy-4-*n*-pentylphenol, 2,6-diethoxy-4-methylphenol, 2,6-diethoxy-4-ethylphenol, 2,6-diethoxy-4-*n*-propylphenol, 4-*n*-butyl-2,6-diethoxyphenol, 2,6-diethoxy-4-*n*-pentylphenol)

In various aspects, the raw reaction product mixture contains less than about 10 wt% residual substrate, more preferably less than about 5 wt% residual substrate and even more preferably less than about 2.5 wt% residual substrate.

### dimers

As used herein, the terms "dimer", "dimers" and "dimeric" refer to m,m'-coupled dimers, o,m'-coupled dimers and/or o,o'-coupled dimers. The terms "isomer", "isomers" and "isomeric" refer only to o,m'-coupled dimers and/or o,o'-coupled dimers.

In various aspects, the raw reaction product mixture is comprised of at least about 80 wt% dimers, more preferably at least about 85 wt% dimers and even more preferably at least about 90 wt% dimers. In further aspects, the raw reaction product mixture comprise at least about 65 wt% m,m'-dimers, more preferably at least about 70 wt% m,m'-dimers and even more preferably at least about 80 wt% m,m'-dimers. In even further aspects, the raw reaction product mixture comprises less than about 15 wt% isomers, more preferably less than about 12 wt% isomers and even more preferably less than about 10 wt% isomers.

### oligomers

As used herein, the terms "oligomer", "oligomers" and "oligomeric" refer to higher condensation (by)products (such as trimers, tetramers, and the like) originating from consecutive over-condensation (i.e. oligomerisation) of dimers.

In various aspects, the raw reaction product mixture comprises less than about 20 wt% isomers, more preferably less than about 15 wt% isomers and even more preferably less than about 10 wt% isomers.

### D. PROCESS OF PURIFICATION

As used herein, "precipitation" is defined as the sedimentation of a solid material (a precipitate) from a liquid solution in which the material is present in amounts greater than its solubility in the liquid (IUPAC Compendium of Chemical Technology). In general, precipitation is a process which happens (homogeneously) throughout the entire solution rather quickly and results in something opaque, cloudy and/or ill-defined.

As used herein, "crystallisation" is defined as the formation of a crystalline solid from a solution, melt vapour or a different solid phase, generally by the lowering of the temperature or by evaporation of a solvent (IUPAC Compendium of Chemical Technology). In general, crystallisation is a process which happens (heterogeneously) at interface of the solution and container rather slowly and results in something with a defined crystal structure.

As used herein, "high purity" is defined as a purity equal or higher than 99.0 wt%, more preferably equal or higher than 99.5 wt% as analysed by melting point measurement (via differential scanning calorimetry), gas chromatography, gel-permeation/size exclusion chromatography, high-pressure liquid chromatography and/or thermogravimetric analysis, and/or combinations thereof.

As used herein, the term "highly pure *meta,meta'-coupled* bis(4-alkylphenol) derivatives" refers to a highly pure meta,meta'-coupled bis(4-alkylphenol) derivative substantially free of impurity comprising the residual substrate, isomeric and/or oligomeric impurity in an amount of less than about 0.2 area-% as measured by gas chromatography or gel-permeation/size exclusion chromatography. Specifically, the meta,meta'-coupled bis(4-alkylphenol) derivative as disclosed herein, contains less than about 0.1 area-%, more specifically less than about 0.05 area-%, still more specifically less than about 0.02 area-% of the impurities.

In another embodiment, the highly pure meta,meta'-coupled bis(4-alkylphenol) derivative disclosed herein has a total purity of greater than about 99 wt%, specifically greater than about 99.5 wt%, more specifically greater than about 99.9 wt%, and most specifically greater than about 99.95 wt% as analysed by melting point measurement (via differential scanning calorimetry), gas chromatography, gel-permeation/size exclusion chromatography, high-pressure liquid chromatography and/or thermogravimetric analysis, and/or combinations thereof. For example, the purity of the meta,meta'-coupled bis(4-alkylphenol) derivative is about 99 wt%, to about 99.9 wt%, or about 99.5 wt% to about 99.99 wt%.

In another embodiment, the meta,meta'-coupled bis(4-alkylphenol) derivative disclosed herein is essentially free of one, or more, of the residual substrate, isomeric or oligomeric impurities. In yet another embodiment, the highly pure meta,meta'-coupled bis(4-alkylphenol) derivative is for instance a highly pure meta,meta'-coupled bis(4-alkylguaiacol) or highly pure *meta*,*meta*'-coupled bis(4-alkylsyringol) derivative

According to the invention, the selective (re)crystallization is a gradual evaporative crystallization process. The selective (re)crystallization is done by using a mixture of non-solvent alkane and solvent.

As used herein, the terms "slow evaporation", "gradual evaporation" and/or "slow gradual evaporation" are defined as an evaporation rate at which the solution of crystallization is maintained at low supersaturation (i.e. conditions in which crystal growth rate exceeds nucleation rate) as indicated by the optical transparency of this solution. Exceeding this specific evaporation rate will result in an opaque, cloudy and non-optical transparent solution (cf. precipitation).

As used herein, the terms "solution of crystallization" and "solution of raw reaction product mixture" are defined and can be used interchangeably as the solvent and/or anti-solvent system in which the raw reaction product mixture is dissolved.

Without further elaboration, it is believed that one skilled in the art can, using the description herein, utilize the present disclosure.

While aspects of the present disclosure can be described and claimed in a particular statutory class, such as the system statutory class, this is for convenience only and one of skill in the art will understand that each aspect of the present disclosure can be described and claimed in any statutory class. Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

### E. USES OF HIGHLY PURE META,META'-COUPLED BIS(4-ALKYLPHENOL) DERIVATIVES

In various aspect the disclosed highly pure bis(4-alkylphenols) derivatives are valuable precursors for making renewable thermoplastics, (high temperature) resins/thermosets, bio-derived C₁₅-C₁₉fuel additives, antioxidants, UV-stabilizers, plasticizers, soluble n-type perylene copolymers and/or positive working photoresists.

In some aspects, these highly pure bis(4-alkylphenol) derivative, being a polyphenol, is converted into a thermoplastic and/or (high temperature) resin/thermoset composition which comprises the bis(4-alkylphenol) derivative, a polyphenol co-monomer, and/or mixtures and/or combinations thereof. Other aspects of the invention further include composites.

Further aspects include converting the polyphenols by reaction with reagents including phosgene, diphosgene (DP), triphosgene (TP), carbonyldiimidazole (CDI), disuccinimidyl carbonate (DSC), diphenylcarbonate, other carbonates, bis(4-chlorophenyl) sulfone, other functionalized sulfones, diacid chlorides, phtalic acids, formaldehyde, other aldehydes, (meth)acryloyl chloride or (meth)acrylic anhydride, dialkoxy methane and epichlorohydrin to produce thermoplastics or thermosetting resins selected from the group consisting of polycarbonates, polyiminocarbonates, polyurethanes, polysulfones (PSf), polyether sulfones (PESf), polyether ether ketones (PEEK), polyester, polyester-styrene polymers, polyacetals, alkylphenolics, polyarylates, cyanate ester resins, epoxy resins, benzoxazine resins, phenolic resins, bismaleimide resins, polyphenylene resins, vinyl ester resins and other endcapped resins (high temperature thermosetting resins) and any combination thereof.

In embodiments, thermosetting resins are combined with the thermoplastics or resins, fiber, or other support materials and are thermally cured either with or without a catalyst to fabricate composite materials.

In some aspects, these bis(4-alkylphenol) derivatives can undergo hydrodeoxygenation (HDO) to produce C₁₅-C₁₉ fuel additives which can be introduced into the heavy fuel fraction in diesel (C₁₀-C₂₂) to improve physicochemical properties and/or serve as a renewable carbon source (Deneyer et al., Curr. Opin. Chem. Biol., 2015, 29, 40-48).

In some aspects, these bis(4-alkylphenol) derivatives can be employed as antioxidants, UV-stabilizers and/or plasticizers, and/or mixtures and/or combinations thereof. The phenolic OH group plays a vital role toward imparting antioxidant characteristics, such as stabilization of polyolefins, like polyethylene and isotactic polypropylene (Pospíšil *et al., Eur. Polym. J.,* 1971, 7, 33-40), due to the potent scavenging activity on aggressive OH radicals and the efficient H-atom donor properties inducing free radical quenching (Torres et al., Food Chem., 2007, 103, 55-61; Ambrogi et al., Biomacromolecules, 2014, 15, 302-310; Reano et al., Green Chem., 2016).

In some aspects, these bis(4-alkylphenol) derivatives can be employed as positive working photoresist and/or photographic material (Inatomi et al., 1995, JP. Patent 07089888 A 19950404)

In some aspects, these bis(4-alkylphenol) derivatives can be applied in organic thin film transistors having solution-processed n-type copolymers as the semi-conductor material. The bio-derived bis(4-alkylphenol) comonomers have flexible solubilizing groups that aid in solubilizing the resulting perylene copolymers and in forming planarizing films of these copolymers (Baca et al., 2015, U.S. Patent 9,082,983 B1).

### E. EXAMPLES

The following examples are put forth so as to provide those of ordinary skill in the art with a complete disclosure and description of how the compounds, articles, devices and/or methods claimed herein are made and evaluated, and are intended to be purely exemplary and are not intended to limit the disclosure. Efforts have been made to ensure accuracy with respect to numbers (e.g. amounts, temperature, etc.), but some errors and deviations should be accounted for. Unless indicated otherwise, parts are parts by weight, temperature is in °C or is at ambient temperature, and pressure is at or near atmospheric. Unless indicated otherwise, percentages referring to composition are in terms of wt %.

There are numerous variations and combinations of reaction conditions, e.g., component concentrations, desired solvents, solvent mixtures, temperatures, pressures and other reaction ranges and conditions that can be used to optimize the product purity and yield obtained from the described process. Only reasonable and routine experimentation will be required to optimize such process conditions.

### 1. GENERAL METHODS AND MATERIALS

All materials and reagents were used as received from the supplier unless otherwise indicated. All chemicals were A.R. grade and other sources can be used besides the ones indicated below.

Analysing mixtures of structurally resembling monomers, dimers and higher oligomers is not straightforward. Thereto, gas chromatography (GC) and gel permeation/size exclusion chromatography (GPC/SEC) were adapted for this analysis and compared. Due to a high chemical similarity among the reagent and products, the dimer yield and monomer conversion can only be measured accurately by GC. Due to the limited volatility of (derivatized) higher oligomers in GC, additional GPC/SEC measurements were performed to confirm their absence/presence.

GC analysis was performed on a Hewlett-Packard (HP) 5890 with a CP-SIL 5CB WCOT fused silica column (30 m x 0.32 mm, film thickness of 1.0 µm), equipped with an FID detector (310 °C) and ChemStation software. The injection port and initial oven temperatures were 300 and 35 °C respectively. This temperature was held for 4 min, increased to 300 °C at 10 °C.min⁻¹, and held there for 40 min. Prior to GC analysis, the samples were derivatized via trimethylsilylation with *N*-methyl-*N*-(trimethylsilyl) trifluoroacetamide (MSTFA). In a typical sample preparation, the dried organic phase (30 mg), together with 10 mg guaiacol (Sigma-Aldrich, ≥98 %; standard 1a) or syringol (Sigma-Aldrich, >99 %; standard 1b) and 10 mg 4,4'-methylenediphenol (TCI Europe, >99 %; standard 2) as external standards, were accurately weighed in a glass vial and homogeneously mixed with 100 µL of anhydrous pyridine (Sigma-Aldrich, 99.8%) and 250 µL. of MSTFA (Sigma-Aldrich, ≥98.5 %). To guarantee a complete derivatization with MSTFA, the samples were heated for 15 min at 80 °C and subsequently diluted with 1 mL acetonitrile (Acros, 99.9+ %). One µl of the sample was injected at a split ratio of 1:100. This GC methodology allows to separate *m*,*m'*-, *o*,*m*'-and *o*,*o*'-isomers and gives an indication of the presence of higher oligomers (e.g. trimers) which only partially evaporate. Quantification of mono- and dimers was performed by calibration of the pure (isolated) reagent/product against the applied external standards.

Idendification of dimeric GC signals was accomplished by gas chromatography-mass spectrometry (GC-MS) on a Agilent 6890 series with a HP1-MS capillary column equipped with an Agilent 5973 series mass spectroscopy detector (El, 70 eV ionisation energy).

To get more insight into the presence, type and (relative) amount of higher oligomers, the distribution of the molar mass of the dried organic phase was investigated using GPC/SEC. Therefore, a sample was solubilized in THF (^{~}10 mg.mL⁻¹; Sigma-Aldrich, >99%) and subsequently filtered with a 0.2 µm PTFE membrane to remove any particulate matter to prevent plugging of the columns. GPC/SEC analysis was performed on a Waters e2695 Separations Module with a pre-column and a Varian M-Gel column (3 µm, mixed), equipped with a Waters 2988 Photodiode array detector (UV detection at 280 nm), Empower software and using THF as the mobile phase (1 mL.min⁻¹ ) at 40 °C. Together with GC, melting point determination and thermogravimetric analysis (TGA), this GPC/SEC analysis was conducted as a means to validate the purity *(i.e.* absence of higher oligomers). In contrast to ¹H-NMR, which provides a number-average oligomer length, GPC/SEC analysis allows to distinguish all oligomers encountered.

Liquid-phase ¹H and ¹³C nuclear magnetic resonance (NMR) spectra were acquired on Bruker Avance instruments (300 and 400 MHz) with automated samplers. The chemical shifts (δ) are reported in parts per million (ppm) referenced to tetramethylsilane (¹H) or the internal NMR solvent signals (¹³C). In a typical sample preparation, a dried sample (^{~}10 mg for ¹H and ^{~}50 mg for ¹³C) is homogeneously dissolved in 650-750 µL of deuterated chloroform (CDCl₃ and transferred to a NMR tube. Fourier-transform infrared (FT-IR) spectra of dried KBr pellets, premixed with pure product (1 wt%), were recorded in vacuo on a Bruker IFS 66v/S instrument.

TGA was performed while heating under N₂ atmosphere using a TA Instruments TGA Q500. About 10 mg of the dried sample was heated at 10 °C.min⁻¹ to 750 °C and kept isothermal for 15 min at a flow rate of 20 mL.min⁻¹. Melting points (*T*ₘ) of the crystals were determined using sealed glass capillaries in a Stuart Scientific SMP3 melting point apparatus and confirmed by differential scanning calorimetry (DSC) on a TA Instruments DSC Q2000 by cycling between 40 and 180 °C at heating/cooling rates of 10 °C.min⁻¹ under N₂ atmosphere.

### a. REFERENCE EXAMPLE 1

4.97 g (36 mmol) 2-methoxy-4-methylphenol (Sigma-Aldrich, ≥98%), 1.46 g (18 mmol) formaldehyde (Sigma-Aldrich, 37 wt%) and 50 mL (2.5 M in H₂O) concentrated HCl (Fisher Scientific, 37 wt%) were placed in a 100 mL Duran^{®} laboratory bottle with screw cap, as well as a magnetic stirring bar (45 mm). At the start of the reaction, the closed bottle is partially (2/3) submerged in a pre-heated stirred temperature-controlled oil bath. The bath is held at 100 °C under continuous stirring (1000 rpm) for 6 hours. Once back at room temperature, the aqueous acidic phase was decanted, the yellowish-green organic (solid) phase dissolved in anhydrous acetone (Sigma-Aldrich, ≥99.9 %) and dried with anhydrous MgSO₄ (Sigma-Aldrich, ≥99.5 %). After filtration of the dried organic phase, n-heptane was carefully added as non-solvent until the solution comes close to the point of supersaturation, but without inducing precipitation. Crystallization of 5,5'-methylenebis(2-methoxy-4-methylphenol) was induced by gradual evaporation of a transparent acetone/n-heptane solution. Highly pure (≥99.0 %) crystals were harvested by filtration and dried in vacuo (25 °C, ~2 mbar) overnight (see Fig. 1A, 2A, 3A and 4A). The mother liquor was left enriched with residual substrate, isomers and oligomers.

**Yield:** 3.68 g (71 %). **M.p.** (powder) 138-139 °C; m.p. (crystals) 140 °C; **¹H NMR** (300 MHz, CDCl₃, 25 °C, TMS): δ_{H} = 2.18 (s, 6H; -ArC*H*₃), 3.69 (s, 2H; -ArC*H*₂Ar-), 3.86 (s, 6H; -OC*H*₃), 5.35 (s, 2H; -ArOH), 6.49 (s, 2H; -o-ArH), 6.68 ppm (s, 2H; -m-ArH); **¹³C NMR** (300 MHz, CDCl₃, 25 °C, TMS): δ_{C} = 144.6, 143.5, 131.4, 127.7, 115.6, 112.9, 56.0, 35.5 and 19.1 ppm; **MS** (70 eV, El): *m*/*z* (%): 289 (19) [*M^{+•}*+^{•}H], 288 (100) [*M^{+•}*], 273 (33) [*M^{+•}*-^{•}CH₃], 151 (24) [*M^{+•}*-^{•}C₈H₉O₂], 150 (75) [*M^{+•}*-C₈H₁₀O₂]; **FTIR** (KBr): *ṽ*ₘₐₓ = 1009 (C-O), 1024 (C-O), 1272 (C-O), 1292 (C-O), 1510 (arom. C=C), 2841 (aliph. C-H), 2910 (aliph. C-H), 2995 (arom. C-H), 3032 (arom. C-H), 3058 (arom. C-H), 3473 (polymeric O-H) and 3529 cm⁻¹ (monomeric O-H)

### b. REFERENCE EXAMPLE 2

5.48 g (36 mmol) 4-ethyl-2-methoxy-phenol (Sigma-Aldrich, ≥98%), 1.46 g (18 mmol) formaldehyde (Sigma-Aldrich, 37 wt%) and 50 mL (2.5 M in H₂O) concentrated HCl (Fisher Scientific, 37 wt%) were placed in a 100 mL Duran^{®} laboratory bottle with screw cap, as well as a magnetic stirring bar (45 mm). At the start of the reaction, the closed bottle is partially (2/3) submerged in a pre-heated stirred temperature-controlled oil bath. The bath is held at 100 °C under continuous stirring (1000 rpm) for 6 hours. Once back at room temperature, the aqueous acidic phase was decanted, the yellowish-orange organic (oily) phase dissolved in anhydrous diethyl ether (Sigma-Aldrich, ≥99 %) and dried with anhydrous MgSO₄ (Sigma-Aldrich, ≥99.5 %). After filtration of the dried organic phase, n-heptane was carefully added as non-solvent until the solution comes close to the point of supersaturation, but without inducing precipitation. Crystallization of 5,5'-methylenebis(4-ethyl-2-methoxyphenol) was induced by gradual evaporation of a transparent diethyl ether/n-heptane solution. Highly pure (≥99.0 %) crystals were harvested by filtration and dried in vacuo (25 °C, _{~}2 mbar) overnight (see Fig. 1B, 2B, 3B and 4B). The mother liquor was left enriched with residual substrate, isomers and oligomers.

**Yield:** 3.90 g (69 %). **M.p.** (powder) 128-129 °C; m.p. (crystals) 129-130 °C; **¹H NMR** (300 MHz, CDCl₃, 25 °C, TMS): δ_{H} = 1.18 (t, ³*J*(H,H)= 7.5 Hz, 6H; -CH₂C*H*₃), 2.54 (q, ³*J*(H,H)= 7.5 Hz, 4H; - C*H*₂CH₃), 3.79 (s, 2H; -ArC*H*₂Ar-), 3.88 (s, 6H; -OC*H*₃), 5.36 (s, 2H; -ArOH), 6.49 (s, 2H; -o-ArH), 6.70 ppm (s, 2H; -*m*-ArH); **¹³C NMR** (300 MHz, CDCl₃, 25 °C, TMS): δ_{C} = 145.0, 143.7, 133.9, 131.2, 116.1, 111.2, 56.2, 34.2, 25.7 and 15.4 ppm; **MS** (70 eV, El): *m*/*z* (%): 317 (13) [*M*^{*+*•}+^{•}H], 316 (61) [*M^{+•}*], 287 (11) [*M^{+•}*-^{•}C₂H₅], 165 (19) [*M^{+•}*-^{•}C₉H₁₁O₂], 164 (100) [*M^{+•}*-C₉H₁₂O₂], 149 (15); **FTIR** (KBr): *ṽ*ₘₐₓ = 1014 (C-O), 1273 (C-O), 1290 (C-O), 1510 (arom. C=C), 2844 (aliph. C-H), 2871 (aliph. C-H), 2931 (aliph. C-H), 2964 (aliph. C-H), 3003 (arom. C-H), 3465 (polymeric O-H) and 3529 cm⁻¹ (monomeric O-H)

### c. REFERENCE EXAMPLE 3

5.98 g (36 mmol) 2-methoxy-4-propylphenol (Sigma-Aldrich, 99+%), 1.46 g (18 mmol) formaldehyde (Sigma-Aldrich, 37 wt%) and 50 mL (2.5 M in H₂O) concentrated HCl (Fisher Scientific, 37 wt%) were placed in a 100 mL Duran^{®} laboratory bottle with screw cap, as well as a magnetic stirring bar (45 mm). At the start of the reaction, the closed bottle is partially (2/3) submerged in a pre-heated stirred temperature-controlled oil bath. The bath is held at 100 °C under continuous stirring (1000 rpm) for 7 hours. Once back at room temperature, the aqueous acidic phase was decanted, the yellowish-red organic (oily) phase dissolved in anhydrous diethyl ether (Sigma-Aldrich, ≥99 %) and dried with anhydrous MgSO₄ (Sigma-Aldrich, ≥99.5 %). After filtration of dried the organic phase, n-heptane was carefully added as non-solvent until the solution comes close to the point of supersaturation, but without inducing precipitation. Crystallization of 5,5'-methylenebis(2-methoxy-4-n-propylphenol) was induced by gradual evaporation of a transparent diethyl ether/n-heptane solution. Highly pure (≥99.0 %) crystals were harvested by filtration and dried in vacuo (25 °C, ~2 mbar) overnight (see Fig. 1C, 2C and 3C). The mother liquor was left enriched with residual substrate, isomers and oligomers.

**Yield:** 3.52 g (57 %). **M.p.** (powder) 78-79 °C; m.p. (crystals) 81-82 °C; **¹H NMR** (300 MHz, CDCl₃, 25 °C, TMS): δ_{H} = 0.95 (t, ³*J*(H,H)= 7.2 Hz, 6H; -CH₂C*H*₃), 1.57 (sex, ³*J*(H,H)= 7.5 Hz, 4H; - CH₂C*H*₂CH₃), 2.48 (t, ³*J*(H,H)= 7.8 Hz, 4H; -ArC*H*₂CH₂-), 3.78 (s, 2H; -ArC*H*₂Ar-), 3.87 (s, 6H; - OC*H*₃), 5.35 (bs, 2H; -ArOH), 6.47 (s, 2H; -o-ArH), 6.67 ppm (s, 2H; -m-ArH); **¹³C NMR** (300 MHz, CDCl₃, 25 °C, TMS): δ_{C} = 144.7, 143.5, 132.4, 131.4, 116.0, 112.0, 56.3, 35.2, 34.4, 24.6 and 14.5 ppm; **MS** (70 eV, El): *m*/*z* (%): 345 (15) [*M*^{*+*•}+^{•}H], 344 (65) [*M^{+•}*], 315 (2) [*M*^{+•}-^{•}C₂H₅], 301 (9) [*M*^{+•}-^{•}C₃H₇], 179 (15) [*M*^{*+*•}-^{•}C₁₀H₁₃O₂], 178 (100) [*M^{+•}*-C₁₀H₁₄O₂]; **FTIR** (KBr): *ṽ*ₘₐₓ = 1018 (CO), 1279 (C-O), 1514 (arom. C=C), 2842 (aliph. C-H), 2868 (aliph. C-H), 2927 (aliph. C-H), 2954 (aliph. C-H), 3012 (arom. C-H) and 3257 (polymeric O-H), 3332 cm⁻¹ (monomeric O-H)

### d. REFERENCE EXAMPLE 4

6.05 g (36 mmol) 2,6-dimethoxy-4-methylphenol (Alfa Aesar, 97 %), 1.46 g (18 mmol) formaldehyde (Sigma-Aldrich, 37 wt%) and 50 mL (2.5 M in H₂O) concentrated HCl (Fisher Scientific, 37 wt%) were placed in a 100 mL Duran^{®} laboratory bottle with screw cap, as well as a magnetic stirring bar (45 mm). At the start of the reaction, the closed bottle is partially (2/3) submerged in a pre-heated stirred temperature-controlled oil bath. The bath is held at 100 °C under continuous stirring (1000 rpm) for 3 hours. Once back at room temperature, the aqueous acidic phase was decanted, the yellowish-white organic (solid) phase dissolved in anhydrous acetone (Sigma-Aldrich, ≥99.9 %) and dried with anhydrous MgSO₄ (Sigma-Aldrich, ≥99.5 %). After filtration of the dried organic phase, n-heptane was carefully added as non-solvent until the solution comes close to the point of supersaturation, but without inducing precipitation. Crystallization of 3,3'-methylenebis(2,6-dimethoxy-4-methylphenol) was induced by gradual evaporation of a transparent acetone/n-heptane solution. Highly pure (≥99.0 %) crystals were harvested by filtration and dried in vacuo (25 °C, ~2 mbar) overnight (see Fig. 1D, 2D and 3D). The mother liquor was left enriched with residual substrate and oligomers.

**Yield:** 4.70 g (75 %). **M.p.** (powder) 144-145 °C; m.p. (crystals) 145 °C; **¹H NMR** (300 MHz, CDCl₃, 25 °C, TMS): δ_{H} = 2.14 (s, 6H; -ArC*H*₃), 3.57 (s, 6H; -2-OC*H*₃), 3.84 (s, 6H; -6-OC*H*₃), 3.93 (s, 2H; -ArC*H*₂Ar-), 5.30 (s, 2H; -ArOH), 6.43 ppm (s, 2H; -m-ArH); **¹³C NMR** (400 MHz, CDCl₃, 25 °C, TMS): δ_{C} = 146.0, 145.2, 136.4, 128.0, 125.9, 108.6, 60.1, 56.1, 24.4 and 20.0 ppm; **MS** (70 eV, El): *m*/*z* (%): 349 (19) [*M^{+•}*+^{•}H], 348 (89) [*M^{•+}*], 181 (80) [*M^{+•}*-^{•}C₉H₁₁O₃], 180 (100) [*M^{+•}*-C₉H₁₂O₃], 167 (18), 165 (25), 151 (27), 137 (21), 121 (14); **FTIR** (KBr): *ṽ*ₘₐₓ = 1057 (C-O), 1078 (C-O), 1298 (C-O), 1315 (C-O), 1502 (arom. C=C), 2837 (aliph. C-H), 2910 (aliph. C-H), 2935 (aliph. C-H), 2947 (aliph. C-H), 2966 (aliph. C-H), 3296 (polymeric O-H) and 3408 cm⁻¹ (monomeric O-H)

### e. REFERENCE EXAMPLE 5

For the catalytic hydrogenolysis experiment, pre-extracted birch wood chips (45 g; particle size 1.5-10 mm; 20.6 wt% Klason lignin), catalyst powder (4.5 g Ru/C; Sigma-Aldrich, 5 wt%) and methanol (240 mL; Sigma-Aldrich, >99%) were loaded into a 600 mL stainless steel batch reactor (Parr Instruments & Co.). The reactor was sealed, flushed threefold with N₂, and pressurized with H₂ (3 MPa at room temperature). Subsequently, the reaction mixture was stirred (750 rpm) and heated to 235 °C (-15 °C.min⁻¹). When the reaction temperature was reached, the temperature was kept constant for 3 h after which the reactor was cooled and depressurized at room temperature. Afterwards, the reactor contents were quantitatively collected by washing extensively with methanol followed by a filtration step to separate the solid residue (pulp and catalyst) from the liquid product mixture.

After reductive processing, the liquid and solid reactor contents were quantitatively collected and separated from each other over a glass fritted filter. Subsequently, the methanol was evaporated. In this way a light-brown oil was obtained, which was subjected to a threefold liquid-liquid extraction using dichloromethane (DCM; Sigma-Aldrich, >99 %) and water to separate the soluble lignin products from sugar-derived products or other polar components. Finally the DCM-extracted phase was dried to obtain the 'lignin oil' (8 - 8.5 g). This lignin oil was subjected to a threefold reflux extraction with n-hexane (Sigma-Aldrich, ≥95%) and the extract was distilled *in vacuo* to obtain a transparent yellow oil (4 -4.5 g) rich in 2,6-dimethoxy-4-n-propylphenol. The product was further purified by column chromatography (25 % v/v acetone in n-heptane) on silica gel (Sigma-Aldrich, pore size 60 Å, 70-230 mesh, 63-200 µm).

**Yield:** 2.31 g (25 % on lignin basis). **¹H NMR** (300 MHz, CDCl₃, 25 °C, TMS): δ_{H} = 0.94 (t, ³*J*(H,H)= 7.3 Hz, 3H; -CH₂C*H*₃), 1.62 (sex, ³*J*(H,H)= 7.3 Hz, 2H; -CH₂C*H*₂CH₃), 2.51 (t, ³*J*(H,H)= 7.3 Hz, 2H; - ArC*H*₂CH₂-), 3.88 (s, 6H; -OC*H*₃), 5.36 (s, 1H; -ArOH) and 6.40 ppm (s, 2H; -m-ArH); **¹³C NMR** (400 MHz, CDCl₃, 25 °C, TMS): δ_{C} = 146.9, 133.9, 132.7, 105.1, 56.3, 38.4, 25.0 and 13.9 ppm; **MS** (70 eV, El): *m*/*z* (%): 196 (36) [*M^{+•}*], 167 (100) [*M*^{+•}-^{•}CH₂CH₃]

7.06 g (36 mmol) 2,6-dimethoxy-4-n-propylphenol (≥98 %), 1.46 g (18 mmol) formaldehyde (Sigma-Aldrich, 37 wt%) and 50 mL (2.5 M in H₂O) concentrated HCl (Fisher Scientific, 37 wt%) were placed in a 100 mL Duran^{®} laboratory bottle with screw cap, as well as a magnetic stirring bar (45 mm). At the start of the reaction, the closed bottle is partially (2/3) submerged in a pre-heated stirred temperature-controlled oil bath. The bath is held at 100 °C under continuous stirring (1000 rpm) for 5 hours. Once back at room temperature, the aqueous acidic phase was decanted, the orange-red organic (oily) phase dissolved in anhydrous diethyl ether (Sigma-Aldrich, ≥99.9 %) and dried with anhydrous MgSO₄ (Sigma-Aldrich, ≥99.5 %). After filtration of the dried organic phase, *n*-heptane was carefully added as non-solvent until the solution comes close to the point of supersaturation, but without inducing precipitation. Crystallization of 3,3'-methylenebis(2,6-dimethoxy-4-*n*-propylphenol) was induced by gradual evaporation of a transparent diethyl ether/*n*-heptane solution. Highly pure (≥99.0 %) crystals were harvested by filtration and dried in vacuo (25 °C, ~2 mbar) overnight (see Fig. 1E, 2E and 3E). The mother liquor was left enriched with residual substrate and oligomers.

**Yield:** 4.36 g (60 %). **M.p.** (powder) 102-103 °C; m.p. (crystals) 103-104 °C; **¹H NMR** (300 MHz, CDCl₃, 25 °C, TMS): δ_{H} = 0.86 (t, ³*J*(H,H)= 7.3 Hz, 6H; -CH₂C*H*₃), 1.34 (sex, ³*J*(H,H)= 7.5 Hz, 4H; - CH₂C*H*₂CH₃), 2.54 (t, ³*J*(H,H)= 7.9 Hz, 4H; -ArC*H*₂CH₂-), 3.51 (s, 6H; -2-OC*H*₃), 3.85 (s, 6H; -6-OC*H*₃), 3.94 (s, 2H; -ArC*H*₂Ar-), 5.30 (s, 2H; -ArOH), 6.45 ppm (s, 2H; -m-ArH); **¹³C NMR** (400 MHz, CDCl₃, 25 °C, TMS): δ_{C} = 145.9, 145.4, 136.5, 132.8, 126.2, 107.8, 60.0, 56.1, 35.5, 24.5, 23.7 and 14.2 ppm; **MS** (70 eV, El): *m*/*z* (%): 405 (10) [*M^{+•}*+^{•}H], 404 (39) [*M^{+•}*], 209 (55) [*M^{+•}*-^{•}C₉H₁₁O₃], 208 (100) [*M^{+•}*-C₉H₁₂O₃], 195 (14), 179 (24), 167 (14); **FTIR** (KBr): *ṽ*ₘₐₓ = 1026 (C-O), 1252 (C-O), 1311 (C-O), 1500 (arom. C=C), 2835 (aliph. C-H), 2866 (aliph. C-H), 2931 (aliph. C-H), 2947 (aliph. C-H), 2962 (aliph. C-H), 3332 (polymeric O-H) and 3545 cm⁻¹ (monomeric O-H)

### f. COUNTER EXAMPLE 1

According to the procedure reported by Meylemans *et al.* (2012), 5.00 g (36.2 mmol) 2-methoxy-4-methylphenol (Sigma-Aldrich, ≥98%) and 1.47 (18.1 mmol) formaldehyde (Sigma-Aldrich, 37 wt%) were diluted with deionized water (40 mL) in a 100 mL round-bottom flask. Concentrated HCl (10 mL; Fisher Scientific, 37 wt%) was slowly added, and the reaction was heated to reflux under N₂ and magnetically stirred for 3 hours. A yellowish-green precipitate formed, the aqueous phase was decanted, and the organic precipitate washed with 10% ethanol solution. Subsequently, the organic phase was dissolved in anhydrous diethyl ether (Sigma-Aldrich, ≥99.9 %, 10 mL) and dried with anhydrous MgSO₄ (Sigma-Aldrich, ≥99.5 %). An impure semi-crystalline yellowish-white powder was obtained by precipitation from ice-cooled n-heptane (100 mL) in 53 wt% containing isomeric and oligomeric impurities (see Fig. 1A, 2A, 3A and 4A). The melting point of this precipitate lies at 132-133 °C (as determined by DSC), which agrees with the melting-range reported by Meylemans *et al.* (2012), being 131-134 °C, but is significantly lower than the melting point (138-139 °C; see REFERENCE EXAMPLE 1) of analogous compounds resulting from our process.

### g. COUNTER EXAMPLE 2

According to the procedure reported by Meylemans *et al.* (2012), 5.48 g (36 mmol) 4-ethyl-2-methoxyphenol (Sigma-Aldrich, ≥98%) and 1.46 (18 mmol) formaldehyde (Sigma-Aldrich, 37 wt%) were diluted with deionized water (40 mL) in a 100 mL round-bottom flask. Concentrated HCl (10 mL; Fisher Scientific, 37 wt%) was slowly added, and the reaction was heated to reflux under N₂ and magnetically stirred for 5 hours. A yellowish-orange oil resulted, the aqueous phase was decanted, and the organic oil washed with 10% ethanol solution. Subsequently, the organic phase was dissolved in anhydrous diethyl ether (Sigma-Aldrich, ≥99.9 %, 10 mL) and dried with anhydrous MgSO₄ (Sigma-Aldrich, ≥99.5 %). An impure semi-crystalline yellowish-white powder was obtained by precipitation from ice-cooled n-heptane (100 mL) in 41 wt% containing isomeric and oligomeric impurities (see Fig. 1B, 2B, 3B and 4B). The melting point (as determined by DSC) lies at 123-124 °C, which is significantly lower than the melting point (128-129 °C; see REFERENCE EXAMPLE 2) of analogous compounds resulting from our process.

### g. COUNTER EXAMPLE 3

According to the procedure reported by Meylemans *et al.* (2012), 5.98 g (36 mmol) 2-methoxy-4-propylphenol (Sigma-Aldrich, 99+%) and 1.46 (18 mmol) formaldehyde (Sigma-Aldrich, 37 wt%) were diluted with deionized water (40 mL) in a 100 mL round-bottom flask. Concentrated HCl (10 mL; Fisher Scientific, 37 wt%) was slowly added, and the reaction was heated to reflux under N₂ and magnetically stirred for 7 hours. A yellowish-red oil formed, the aqueous phase was decanted, and the organic oil washed with 10% ethanol solution. Subsequently, the organic phase was dissolved in anhydrous diethyl ether (Sigma-Aldrich, ≥99.9 %, 10 mL) and dried with anhydrous MgSO₄ (Sigma-Aldrich, ≥99.5 %). While gradual evaporation of an diethyl ether/n-heptane solution yielded crystals (melting point 78-79 °C; see REFERENCE EXAMPLE 3), precipitation from ice-cooled n-heptane (50-100 mL) was not possible and yielded a suspension.

### h. COUNTER EXAMPLE 4

According to the procedure reported by Meylemans *et al.* (2012), 6.05 g (36 mmol) 2,6-dimethoxy-4-methylphenol (Alfa Aesar, 97 %) and 1.46 g (18 mmol) formaldehyde (Sigma-Aldrich, 37 wt%) were diluted with deionized water (40 mL) in a 100 mL round-bottom flask. Concentrated HCl (10 mL; Fisher Scientific, 37 wt%) was slowly added, and the reaction was heated to reflux under N₂ and magnetically stirred for 2 hours. A yellowish-white precipitate formed, the aqueous phase was decanted, and the organic oil washed with 10% ethanol solution. Subsequently, the organic phase was dissolved in anhydrous diethyl ether (Sigma-Aldrich, ≥99.9 %, 10 mL) and dried with anhydrous MgSO₄ (Sigma-Aldrich, ≥99.5 %). An impure semi-crystalline off-white powder was obtained by precipitation from ice-cooled n-heptane (100 mL) in 83 wt% containing oligomeric impurities (see Fig. 1D, 2D and 3D).

### i. COUNTER EXAMPLE 5

According to the procedure reported by Meylemans *et al.* (2012), 7.06 g (36 mmol) 2,6-dimethoxy-4-n-propylphenol (≥98 %) and 1.46 (18 mmol) formaldehyde (Sigma-Aldrich, 37 wt%) were diluted with deionized water (40 mL) in a 100 mL round-bottom flask. Concentrated HCl (10 mL; Fisher Scientific, 37 wt%) was slowly added, and the reaction was heated to reflux under N₂ and magnetically stirred for 7 hours. A orange-red oil formed, the aqueous phase was decanted, and the organic oil washed with 10% ethanol solution. Subsequently, the organic phase was dissolved in anhydrous diethyl ether (Sigma-Aldrich, ≥99.9 %, 10 mL) and dried with anhydrous MgSO₄ (Sigma-Aldrich, ≥99.5 %). While gradual evaporation of an diethyl ether/n-heptane solution yielded crystals (melting point 102-103 °C; see REFERENCE EXAMPLE 5), precipitation from ice-cooled *n*-heptane (50-100 mL) was not possible and yielded a suspension.

## Claims

1. A process for the production of aromatic dihydroxy compounds of >97 wt% purity, with a general formula according to (I): 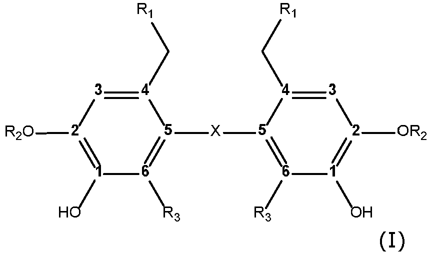
a. wherein X is an alkanediyl C1-C24 organic group;
b. wherein R₁ is independently selected from hydrogen, methyl or ethyl;
c. wherein R₂ is independently selected from methyl or ethyl;
d. wherein R₃ is independently selected from hydrogen, methoxy or ethoxy;
the process comprising:
(1) carrying out a condensation reaction of i) 4-alkylphenol derivative and/or combinations thereof with ii) carbonylic compounds to produce a raw reaction product mixture,
(2) forming crystals of aromatic dihydroxy compounds by gradual evaporation directly of the solution of raw reaction product in a mixture of non-solvent alkane and solvent, whereby the gradual evaporation is at an evaporation rate that prevents precipitation, and
(3) separating said crystals.

2. The process according to claim 1, whereby the 4-alkylphenol derivative comprises 2-alkoxy-4-alkylphenols and 2,6-dialkoxy-4-alkylphenols or whereby the 4-alkylphenol derivative is any one of the group consisting of 2-methoxy-4-n-propylphenol, 2,6-dimethoxy-4-n-propylphenol or whereby the 4-alkylphenol derivative consists of mixtures and/or combinations of 2 or more of said 4-alkylphenol derivatives to yield asymmetric m,m'-bis(4-alkylphenol) derivatives.

3. The process according to claim 1 or 2, whereby the gradual evaporation is at an evaporation rate such to maintain the supersaturation whereby the crystal growth rate exceeds nucleation rate.

4. The process according to claim 1 or 2, whereby the gradual evaporation is at an evaporation rate so that so that the solution remains transparent, for instance the (white) light transparency or pellucidity of the solution remains between 50 and 90%.

5. The process according to claim 1 or 2, whereby the gradual evaporation is at an evaporation rate so that the solution remains translucent, for instance translucency of the solution is maintained between 50 and 90%.

6. The process according to any one of the preceding claims, whereby the gradual evaporation is from raw reaction product in an acetone/alkane solution or in a diethyl ether/alkane solution for instance in an acetone/n-heptane solution or in a diethyl ether/n-heptane solution.

7. The process according to claim 1 or 2, whereby the gradual evaporation is at an evaporation rate that prevents the solution becoming opaque.

8. The process according to any one of claims 1 to 7, whereby the evaporation rate is controlled by any one of the following: a) evaporated substance concentration in the fluid contacting the solution, b) evacuation rate of the fluid contacting said solution, c) inter-molecular forces in the solution, d) pressure on the solution and e) temperature.

9. The process according to any one of claims 1 to 8, whereby the aromatic dihydroxy compounds have a purity of ≥99.0 wt% for instance as determined by the melting point.

10. The process according to any one of claims 1 to 9, whereby the raw reaction product mixture comprises residual substrate, dimers and oligomers.

11. The process according to claim 10, whereby said aromatic dihydroxy compounds are meta,meta'-coupled bis(4-alkylguaiacol) derivatives and whereby said 4-alkylguaiacol is selected from the group consisting of 2-methoxy-4-methylphenol (creosol), 4-ethyl-2-methoxyphenol and 2-methoxy-4-n-propylphenol.

12. The process according to claim 11, whereby said aromatic dihydroxy compounds are meta,meta'-coupled bis(4-alkylsyringol) derivatives and whereby said 4-alkylsyringol is selected from the group consisting of 2,6-dimethoxy-4-methylphenol, 4-ethyl-2,6-dimethoxyphenol and 2,6-dimethoxy-4-n-propylphenol.

13. The process according to any one of claims 1 to 12, whereby the purity is analysed by any one of the following methods, being melting point measurement by differential scanning calorimetry, gas chromatography, high-pressure liquid chromatography and/or gel permeation/size-exclusion chromatography and/or combinations thereof, most preferably by melting point measurement by differential scanning calorimetry.

14. The process according to any one of claims 1 to 12, further comprising utilizing said aromatic dihydroxy compounds as plasticizers and/or UV-stabilizers.

15. The process according to claim 12, further comprising converting said aromatic dihydroxy compounds to polymers.

## Patentansprüche

1. Verfahren für die Herstellung von aromatischen Dihydroxyverbindungen einer Reinheit von >97 Gew.-%, mit einer allgemeinen Formel (I) entsprechend: 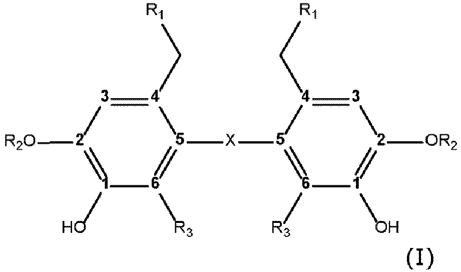
a. wobei X eine organische C1-C24-Alkandiylgruppe ist;
b. wobei R₁ unabhängig unter Wasserstoff, Methyl oder Ethyl ausgewählt ist;
c. wobei R₂ unabhängig unter Methyl oder Ethyl ausgewählt ist;
d. wobei R₃ unabhängig unter Wasserstoff, Methoxy oder Ethoxy ausgewählt ist;
wobei das Verfahren Folgendes umfasst:
(1) Ausführen einer Kondensationsreaktion von i) 4-Alkylphenolderivat und/oder Kombinationen davon mit ii) Carbonylverbindungen, um eine rohe Reaktionsproduktmischung herzustellen,
(2) Bilden von Kristallen aromatischer Dihydroxyverbindungen durch allmähliches direktes Verdampfen der Lösung von rohem Reaktionsprodukt in einer Mischung von Nichtlösungsmittelalkan und Lösungsmittel, wobei das allmähliche Verdampfen mit einer Verdampfungsrate erfolgt, die Ausfällen verhindert, und
(3) Trennen der Kristalle.

2. Verfahren nach Anspruch 1, wobei das 4-Alkylphenolderivat 2-Alkoxy-4-alkylphenole und 2,6-Dialkoxy-4-alkylphenole umfasst oder wobei das 4-Alkylphenolderivat irgendeines ist aus der Gruppe bestehend aus 2-Methoxy-4-n-propylphenol, 2,6-Dimethoxy-4-n-propylphenol oder wobei das 4-Alkylphenolderivat aus Mischungen und/oder Kombinationen von 2 oder mehr der 4-Alkylphenolderivate besteht, um asymmetrische m,m'-Bis(4-alkylphenol)derivate zu ergeben.

3. Verfahren nach Anspruch 1 oder 2, wobei das allmähliche Verdampfen bei einer Verdampfungsrate erfolgt derart, dass die Übersättigung aufrechterhalten wird, wobei die Kristallwachstumsrate die Keimbildungsrate übersteigt.

4. Verfahren nach Anspruch 1 oder 2, wobei das allmähliche Verdampfen bei einer Verdampfungsrate erfolgt, so dass die Lösung transparent bleibt, beispielsweise die (Weiß-) Lichttransparenz oder Klarheit der Lösung zwischen 50 und 90 % bleibt.

5. Verfahren nach Anspruch 1 oder 2, wobei das allmähliche Verdampfen bei einer Verdampfungsrate erfolgt, so dass die Lösung lichtdurchlässig bleibt, beispielsweise die Lichtdurchlässigkeit der Lösung zwischen 50 und 90 % aufrechterhalten wird.

6. Verfahren nach einem der vorhergehenden Ansprüche, wobei das allmähliche Verdampfen aus rohem Reaktionsprodukt in einer Aceton-/Alkanlösung oder in einer Diethylether-/Alkanlösung, beispielsweise in einer Aceton-/n-Heptanlösung oder in einer Diethylether-/n-Heptanlösung erfolgt.

7. Verfahren nach Anspruch 1 oder 2, wobei das allmähliche Verdampfen bei einer Verdampfungsrate erfolgt, die verhindert, dass die Lösung undurchsichtig wird.

8. Verfahren nach einem der Ansprüche 1 bis 7, wobei die Verdampfungsrate durch irgendeines der Folgenden reguliert wird: a) der Konzentration der verdampften Substanz in dem die Lösung kontaktieren Fluid, b) der Verdampfungsrate des die Lösung kontaktierenden Fluids, c) intermolekularen Kräften in der Lösung, d) Druck auf die Lösung und e) Temperatur.

9. Verfahren nach einem der Ansprüche 1 bis 8, wobei die aromatischen Dihydroxyverbindungen eine Reinheit von ≥99,0 Gew.-%, wie beispielsweise durch den Schmelzpunkt bestimmt, aufweisen.

10. Verfahren nach einem der Ansprüche 1 bis 9, wobei die rohe Reaktionsproduktmischung restliches Substrat, Dimere und Oligomere umfasst.

11. Verfahren nach Anspruch 10, wobei die aromatischen Dihydroxyverbindungen meta,meta`-gekoppelte Bis(4-alkylguaiacol)derivate sind und wobei das 4-Alkylguaiacol aus der Gruppe ausgewählt ist bestehend aus 2-Methoxy-4-methylphenol (Creosol), 4-Ethyl-2-methoxyphenol und 2-Methoxy-4-n-propylphenol.

12. Verfahren nach Anspruch 11, wobei die aromatischen Dihydroxyverbindungen meta,meta`-gekoppelte Bis(4-alkylsyringol)derivate sind und wobei das 4-Alkylsyringol aus der Gruppe ausgewählt ist bestehend aus 2,6-Dimethoxy-4-methylphenol, 4-Ethyl-2,6-dimethoxyphenol und 2,6-Dimethoxy-4-n-propylphenol.

13. Verfahren nach einem der Ansprüche 1 bis 12, wobei die Reinheit durch irgendeine der folgenden Methoden analysiert wird, was Schmelzpunktmessbestimmung durch Differential-Scanning-Kalorimetrie, Gaschromatographie, Hochdruckflüssigchromatographie und/oder Gelpermeations-/Größenausschlusschromatographie und/oder Kombinationen davon, am bevorzugtesten durch Schmelzpunktmessbestimmung durch Differential-Scanning-Kalorimetrie ist.

14. Verfahren nach einem der Ansprüche 1 bis 12, ferner Verwenden der aromatischen Dihydroxyverbindungen als Weichmacher und/oder UV-Stabilisatoren umfassend.

15. Verfahren nach Anspruch 12, ferner Umwandeln der aromatischen Dihydroxyverbindungen in Polymere umfassend.

## Revendications

1. Processus de production de composés dihydroxy aromatiques de pureté > 97 % en poids, ayant une formule générale selon (I) : 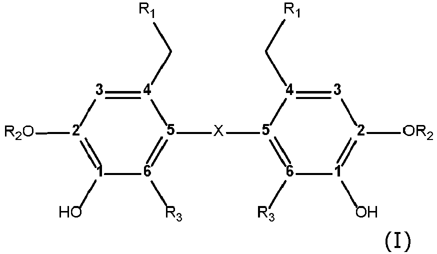
a. dans laquelle X est un groupe organique alcanediyle en C1-C24 ;
b. dans laquelle R₁ est choisi indépendamment parmi l'hydrogène, le méthyle ou l'éthyle ;
c. dans laquelle R₂ est choisi indépendamment parmi le méthyle ou l'éthyle ;
d. dans laquelle R₃ est choisi indépendamment parmi l'hydrogène, le méthoxy ou l'éthoxy ;
le processus comprenant :
(1) l'exécution d'une réaction de condensation de i) un dérivé de 4-alkylphénol et/ou des combinaisons de ce dernier avec ii) des composés carbonylés pour produire un mélange de produit de réaction brut,
(2) la formation de cristaux de composés dihydroxy aromatiques par évaporation graduelle directement de la solution de produit de réaction brut dans un mélange d'alcane non-solvant et de solvant, de sorte que l'évaporation graduelle se fait à un taux d'évaporation qui empêche la précipitation, et
(3) la séparation desdits cristaux.

2. Processus selon la revendication 1, de sorte que le dérivé de 4-alkylphénol comprend des 2-alkoxy-4-alkylphénols et des 2,6-dialkoxy-4-alkylphénols, ou de sorte que le dérivé de 4-alkylphénol est l'un quelconque du groupe constitué de 2-méthoxy-4-n-propylphénol, 2,6-diméthoxy-4-n-propylphénol, ou de sorte que le dérivé de 4-alkylphénol consiste en des mélanges et/ou des combinaisons de 2 ou plus desdits dérivés de 4-alkylphénol pour produire des dérivés m,m'-bis(4-alkylphénol) asymétriques.

3. Processus selon la revendication 1 ou 2, de sorte que l'évaporation graduelle se fait à un taux d'évaporation permettant de maintenir la supersaturation de sorte que le taux de croissance de cristaux dépasse le taux de nucléation.

4. Processus selon la revendication 1 ou 2, de sorte que l'évaporation graduelle se fait à un taux d'évaporation tel que la solution reste transparente, par exemple la transparence à la lumière (blanche) ou la pellucidité de la solution reste entre 50 et 90 %.

5. Processus selon la revendication 1 ou 2, de sorte que l'évaporation graduelle se fait à un taux d'évaporation tel que la solution reste translucide, par exemple la translucidité de la solution est maintenue entre 50 et 90 %.

6. Processus selon l'une quelconque des revendications précédentes, de sorte que l'évaporation graduelle est effectuée à partir de produit de réaction brut dans une solution d'acétone/alcane ou dans une solution d'éther diéthylique/alcane, par exemple dans une solution d'acétone/n-heptane ou dans une solution d'éther diéthylique/n-heptane.

7. Processus selon la revendication 1 ou 2, de sorte que l'évaporation graduelle se fait à un taux d'évaporation qui empêche la solution de devenir opaque.

8. Processus selon l'une quelconque des revendications 1 à 7, de sorte que le taux d'évaporation est régulé par l'un quelconque de ce qui suit : a) concentration de la substance évaporée dans le fluide en contact avec la solution, b) taux d'évacuation du fluide en contact avec ladite solution, c) forces intermoléculaires dans la solution, d) pression sur la solution et e) température.

9. Processus selon l'une quelconque des revendications 1 à 8, de sorte que les composés dihydroxy aromatiques ont une pureté ≥ 99,0 % en poids, par exemple telle que déterminée par le point de fusion.

10. Processus selon l'une quelconque des revendications 1 à 9, de sorte que le mélange de produit de réaction brut comprend un substrat résiduel, des dimères et des oligomères.

11. Processus selon la revendication 10, de sorte que lesdits composés dihydroxy aromatiques sont des dérivés méta,méta'-couplés de bis(4-alkylguaiacol), et de sorte que ledit 4-alkylguaiacol est choisi parmi le groupe constitué de 2-méthoxy-4-méthylphénol (créosol), 4-éthyl-2-méthoxyphénol et 2-méthoxy-4-n-propylphénol.

12. Processus selon la revendication 11, de sorte que lesdits composés dihydroxy aromatiques sont des dérivés méta,méta'-couplés de bis(4-alkylsyringol), et de sorte que ledit 4-alkylsyringol est choisi parmi le groupe constitué de 2,6-diméthoxy-4-méthylphénol, 4-éthyl-2,6-diméthoxyphénol et 2,6-diméthoxy-4-n-propylphénol.

13. Processus selon l'une quelconque des revendications 1 à 12, de sorte que la pureté est analysée par l'un quelconque des procédés suivants : mesure du point de fusion par calorimétrie différentielle à balayage, chromatographie en phase gazeuse, chromatographie liquide à haute pression et/ou chromatographie par perméation de gel/séparation par taille et/ou des combinaisons de ceux-ci, de préférence par mesure du point de fusion par calorimétrie différentielle à balayage.

14. Processus selon l'une quelconque des revendications 1 à 12, comprenant en outre l'utilisation desdits composés dihydroxy aromatiques en tant que plastifiants et/ou stabilisateurs UV.

15. Processus selon la revendication 12, comprenant en outre la conversion desdits composés dihydroxy aromatiques en polymères.
